Europäisches Patentamt

European Patent Office

Office européen des brevets

(11) Veröffentlichungsnummer: **0 368 224**

**A2**

(12) # EUROPÄISCHE PATENTANMELDUNG

(21) Anmeldenummer: 89120551.0

(22) Anmeldetag: 07.11.89

(51) Int. Cl.5: **C12N 15/56, C12N 15/76, C12N 1/21, C12N 9/36, C12Q 1/68, //(C12N1/21, C12R1:465)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4913 und DSM 4914.

(30) Priorität: 10.11.88 DE 3838107
17.03.89 DE 3908766

(43) Veröffentlichungstag der Anmeldung:
**16.05.90 Patentblatt 90/20**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Marquardt, Rüdiger, Dr.**
**Günthersburgallee 69**
**D-6000 Frankfurt am Main 60(DE)**
Erfinder: **Bräu, Barbara, Dr.**
**Johannesallee 8**
**D-6230 Frankfurt am Main 80(DE)**

Erfinder: **Wöhner, Gerhard, Dr.**
**Mühlenkampstrasse 77**
**D-3050 Wunstorf(DE)**
Erfinder: **Präve, Paul, Prof. Dr.**
**Kronberger Weg 7**
**D-6231 Sulzbach (Taunus)(DE)**
Erfinder: **Schlingmann, Merten, Prof. Dr.**
**Schneidhainer Strasse 32a**
**D-6240 Königstein/Taunus(DE)**
Erfinder: **Pühler, Alfred, Prof. Dr.**
**Am Waldschlösschen 2**
**D-4800 Bielefeld(DE)**
Erfinder: **Birr, Elli**
**Wellensiek 90**
**D-4800 Bielefeld(DE)**
Erfinder: **Wohlleben, Wolfgang, Dr.**
**Menzelstrasse 1**
**D-4800 Bielefeld(DE)**
Erfinder: **Aufderheide, Karin**
**Johanniskirchplatz 1a**
**D-4800 Bielefeld(DE)**
Erfinder: **Schneider, Thomas**
**Kösterkamp 14**
**D-4800 Bielefeld(DE)**

(54) Lysozymgen aus Streptomyceten, Verfahren zu seiner Gewinnung und seine Verwendung.

(57) Das Lysozymgen aus Streptomyceten kann mit Hilfe einer Oligonukleotidsonde mit 38 Nukleotiden oder einer Lysozym-defizienten Mutante von Streptomyces coelicolor identifiziert und in S. coelicolor exprimiert werden.

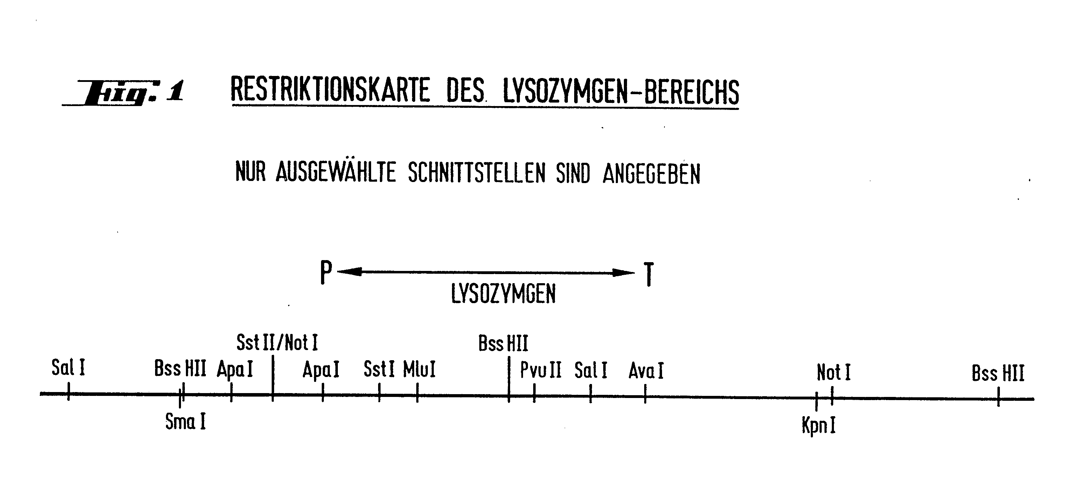

**Fig. 1** RESTRIKTIONSKARTE DES LYSOZYMGEN-BEREICHS

NUR AUSGEWÄHLTE SCHNITTSTELLEN SIND ANGEGEBEN

## Lysozymgen aus Streptomyceten, Verfahren zu seiner Gewinnung und seine Verwendung

Die vorliegende Erfindung betrifft die Klonierung eines Lysozym-Gens aus Streptomyceten, den Transfer dieses Gens in unterschiedliche Mikroorganismen-Stämme und die Herstellung des Genprodukts. Ebenfalls beschrieben wird die Veränderung des Gens durch Austausch der umgebenden Signalsequenzen.

Als Lysozyme werden Enzyme definiert, die als 1,4-ß-N-Acetylmuramidasen die glykosidische Bindung zwischen $C_1$ der N-acetylmuraminsäure und $C_4$ des N-Acetylglucosamins im bakteriellen Peptidoglykan spalten [Jollès, P. und Jollès, J.: Molecular and Cellular Biochemistry 63, 165-189 (1984)]. Lysozyme sind in einer Vielzahl von Organismen gefunden und bearbeitet worden. Für das Lysozym aus Hühnereiweiß ist eine Aminosäuresequenz veröffentlicht und ein Reaktionsmechanismus vorgeschlagen worden (Jollès et al.: Biochim. Biophys. Acta 78, 668-689, 1963; Phillips, D.C.: Sci. Am. 215, 78-90, 1966; als Übersicht siehe Jollès und Jollès wie oben zitiert). Zu den anderen Spezies, in denen Lysozyme nachgewiesen wurden, gehören auch einige Streptomyceten-Stämme, wie Streptomyces labedae (Streptomyces "erythraeus") [.Morita et al., J. Biochem. 83, 893-903, (1978).], Streptomyces griseus [.Ward und Perkins, Biochem. J. 106, 67-76, (1968)], Streptomyces globisporus [Kawata et al. : Agric. Biol. Chem. 47, 1501-1508, (1983)], Streptomyces rutgersensis [.Hayashi et al. : J. Ferment. Technol. 59, 319-323 (1981)] und Streptomyces coelicolor (Müller) (DE 3440735).

Mit der Entwicklung gentechnischer Methoden konnten verschiedene Lysozym-Gene kloniert und im Detail analysiert werden. Hierzu gehören beispielsweise die Gene für das Lysozym aus Hühnereiweiß (Nguyen-Huu et al.: Proc. Natl. Acad. Sci. USA 76, 76 80, 1979) bzw. für das Lysozym des Phagen T4 (Owen et al.: J. Mol. Biol. 105, 229-248, 1983).

Daneben hat man die Methode der chemischen DNA-Synthese verwendet, um Lysozym-Gene neu zu synthetisieren. Verwiesen sei hier auf die Neusynthese des menschlichen Lysozym-Gens (EP 0 181 634; Ikehara et al.: Chem. Pharm. Bull. 34, 2202-2208, 1986) und dessen Expression in Mikroorganismen (EP 0 255 233; Muraki et al.: Agric. Biol. Chem. 49, 2829-2831, 1985; Yoshimura et al.: Biochem. Biophys. Res. Comm. 150, 794 801, 1988). Die neuen Methoden der Gentechnik wurden auch verwendet, um natürlich vorkommende Lysozym-Gene gezielt zu verändern (Perry,L.J. and Wetzel, R.: Science 226, 555-557, 1984; Wetzel et al.: Proc. Natl. Acad. Sci. 85, 401-405, 1988; Muraki et al.: Biochim. Biophys. Acta 916, 66 75, 1987) und damit Aufschluß über Reaktionsmechanismen zu erlangen, bzw. Enzymeigenschaften, wie z.B. thermische Stabilität zu beeinflussen.

Es wurde nun das Lysozym kodierende Gen in Streptomyceten gefunden. Die Erfindung betrifft somit ein Lysozymgen aus Streptomyceten, das dadurch gekennzeichnet ist, daß es

a) mit einer Oligonukleotidsonde der Sequenz 5'-TTC GC (G/C)TAC ATC AAG GC(G/C) AC(G/C) GAG GG(G/C) AC(G/C) AAC TAC AA-3'
hybridisiert und/oder

b) die Lysozym negative Mutante Streptomyces coelicolor DSM 4913 nach Transformation komplementiert.

Das erfindungsgemäße mikrobielle Lysozymgen wird aus Streptomyceten gewonnen, vorzugsweise aus den Stämmen Streptomyces coelicolor (Müller) und Streptomyces labedae, bzw. aus Mutanten und Varianten dieser Stämme. Die Stämme DSM 3030 (S. coelicolor) und DSM 41517 (S.labedae) sind besonders bevorzugt.

Die Gesamt-DNA wird nach gängigen Methoden aus diesen Stämmen isoliert und in Fragmente verschiedener Größe zerteilt, die dann mit Cosmid- oder Plasmid-Vektoren verbunden und in Wirtsstämme transformiert werden können. Alternativ kann eine vorherige Eingrenzung der interessierenden chromosomalen DNA-Fragmente dadurch erfolgen, daß man die verdaute Gesamt-DNA gegen radioaktiv markierte Oligonukleotide hybridisiert. Die Sequenz der eingesetzten Oligonukleotide muß dabei nach den Regeln des genetischen Codes einer zuvor ermittelten Aminosäureabfolge im Lysozym-Protein entsprechen.

Aus entsprechenden Genbanken muß dann der das Lysozymgen enthaltende Wirtsklon identifiziert werden. Die Vorgehensweisen sind abhängig vom verwendeten Wirtsstamm nachstehend beschrieben.

Zur Identifizierung des in E.coli klonierten Lysozymgens ist der Einsatz eines Aktivitätstests wenig sinnvoll, da die Expression von Streptomyceten-Genen mit ihren natürlichen Signalsequenzen in E.coli nur schlecht funktioniert. Die deutliche Ausprägung einer Lysozym-Aktivität in E.coli ist daher nicht zu erwarten und wäre für die Zelle möglicherweise sogar toxisch. Eine Auffindung des gesuchten Klons ist aber über den Einsatz von Oligonukleotid-Sonden nach Erstellung von partiellen Aminosäure-Sequenzen des in Fragmente zerlegten Lysozyms möglich. Aus der jeweiligen Aminosäure-Abfolge ergibt sich dann nach den Regeln des genetischen Codes die zugehörige Abfolge von Nukleotiden auf Ebene der DNA, wobei eine bestimmte Aminosäure von bis zu 6 unterschiedlichen Codons determiniert werden kann.

In den ermittelten Aminosäure-Sequenzen wird nach Bereichen gesucht, in denen Aminosäuren aufeinanderfolgen, die durch eine möglichst geringe Anzahl von Codons auf Ebene der DNA determiniert sein können. Aus diesen Aminosäure-Sequenz-Bereichen werden die Sequenzen von korrespondierenden Oligonukleotiden abgeleitet. Durch die Degeneration des genetischen Codes steigt die Anzahl möglicher Nukleotid-Sequenzen schnell an, so daß man sich in der Regel mit einer Abfolge von rund 20 Nukleotiden begnügt.

Derartige Sonden ergaben zwar in Vorversuchen teilweise recht spezifische Signale, erwiesen sich letztlich für das Auffinden des Gens aber als ungeeignet. Erst die obengenannte Sonde mit 38 Nukleotiden 5'-TTC GC (G/C)TAC ATC AAG GC(G/C) AC(G/C) GAG GG(G/C) AC(G/C) AAC TAC AA-3' ist sowohl in Vorversuchen als auch in den nachfolgend beschriebenen Screening-Verfahren erfolgreich einzusetzen. Aufgrund des hohen G/C Gehalts der Streptomyceten-DNA von über 70 % wurde bei der Synthese der Sonde in der 3. Position der verwendeten Codons nur G oder C eingesetzt. Mit dieser Sonde können nicht nur in der Gesamt-DNA von S.coelicolor (Müller), sondern auch in der Gesamt-DNA von anderen Streptomyceten-Stämmen, z.B. S.labedae, spezifisch hybridisierende DNA-Banden identifiziert werden.

Die Klone einer vorhandenen Plasmid- oder Cosmid-Genbank werden auf unterschiedliche Weise untersucht. Zum einen werden die erhaltenen Klone direkt auf Filtern lysiert und die ausgetretene DNA auf Hybridisierung gegen das eingesetzte radioaktiv markierte Oligonukleotid getestet. Zum anderen werden die erhaltenen Klone einzeln oder in Pools von jeweils 10 Klonen angezüchtet und die enthaltene Plasmid/Cosmid-DNA isoliert. Diese DNA wird dann auf Filter aufgetropft oder nach Agarose-Gelelektrophorese, der wiederum eine Verdauung mit Restriktionsenzymen vorausgehen kann, durch Southern Blotting auf Filter übertragen. Die an das Filter fixierte DNA wird mit dem radioaktiv markierten Oligonukleotid hybridisiert. Nach Autoradiographie zeigt eine Schwärzung des Films die Stellen an, wo das Oligonukleotid an das Filter gebunden ist. Stammt die korrespondierende Cosmid- oder Plasmid-DNA aus einem Pool von 10 Klonen, so werden die entsprechenden Klone jetzt einzeln aufgearbeitet und die Cosmid- bzw. Plasmid- DNAs einzeln gegen das Oligonukleotid hybridisiert. Die Cosmid- bzw. Plasmid-DNA eines als positiv eingestuften Klons kann dann durch Verdauung mit verschiedenen Restriktionsenzymen in Fragmente gespalten und diese wiederum mit dem Oligonukleotid hybridisiert werden. Auf diese Weise gelingt eine Eingrenzung des DNA-Bereichs, welcher mit dem Oligonukleotid hybridisiert.

Durch Subklonierung dieses DNA-Bereichs in Sequenziervektoren, z.B. in den Sequenziervektor pUC 8 oder den daraus abgeleiteten Sequenziervektor pSVB 26, und anschließende Sequenzierung und Vergleich mit den zuvor ermittelten Aminosäure-Sequenzen kann gezeigt werden, daß es sich bei der isolierten DNA tatsächlich um das gesuchte Gen handelt. Mit Hilfe von Computer-Programmen kann das Strukturgen sowie die 5'- bzw. 3'-Bereiche eindeutig definiert werden (Tabellen 1 und 2). Das Lysozym kodierende 1,2 kb AvaI-Fragment wurde aus den Sequenziervektoren pUC8 oder pSVB 26 in Streptomyceten-Vektoren, z.B. pEB 15 (als DSM 4914 in einer Lysozym-defizienten Mutante bei der Deutschen Sammlung von Mikroorganismen und Zellkulturen unter den Bedingungen des Budapester Vertrags hinterlegt) kloniert. Nach Transformation in Lysozym-Produzenten Stämme, wie z.B. S. coelicolor DSM 3030, wird eine Ausbeutesteigerung um den Faktor 3-5 möglich.

Bei einer Klonierung in Streptomyceten bietet sich zunächst der Stamm S.lividans als Wirtsorganismus an, da dieser Stamm relativ einfach zu bearbeiten und eine Expression des gesuchten Gens in diesem Streptomyceten zu erwarten ist. Zur Anlage einer Genbank wurden sowohl isolierte Gesamt-DNA als auch angereicherte Fragmente aus einem Agarose-Gel mit E.coli/Streptomyceten shuttle-Vektoren bzw. Streptomyceten-Vektoren verbunden. Es konnte allerdings auch bei Austestung von mehr als hunderttausend Transformanden kein das Lysozymgen tragender S.lividans Klon identifiziert werden.

Die Klonierung des Gens in das homologe System ist jedoch möglich. Das erfindungsgemäße Lysozymgen kann demnach durch Komplementierung Lysozym-defizienter Mutanten identifiziert werden. Stabile Mutanten von S. coelicolor DSM 3030, die keine oder eine signifikant verringerte Lysozymproduktion aufweisen, können spontan entstehen oder durch chemische oder physikalische Mutagenese von Sporen, Mycelbruchstücken oder isolierten Protoplasten gewonnen werden. Bevorzugt werden Sporen von S. coelicolor DSM 3030 eingesetzt und mit chemischen Mutagenen, wie z.B. N-methyl-N'-nitro-N- nitrosoguanidin (MNNG), Nitrosomethylharnstoff, Methylmethansulfonat, Ethylmethansulfonat, 4- Nitrochinolin-1-oxid, Hydroxylamin, 5-Bromouracil, Natriumnitrit, Mitomycin C, Ethidiumbromid oder Acriflavin, behandelt oder mit 8-Methoxypsoralen bei anschließender Bestrahlung mit langwelligem UV-Licht (365 nm) umgesetzt. Alternativ können Sporen mit kurzwelligem UV-Licht (254 nm) bestrahlt werden. Die Behandlung mit chemischen Mutagenen, bevorzugt MNNG bzw. die Bestrahlung mit UV-Licht (254 nm) wird bis zu einer Überlebensrate von 0,1 % bis 30 %, bevorzugt 1 % bis 2 % durchgeführt. Die durch UV- Mutagenese von Sporen von S. coelicolor DSM 3030 erhaltene stabile Lysozym-defiziente Mutante, S. coelicolor, ist bei der Deutschen Sammlung von Mikroorganismen und Zellkulturen unter den Bedingungen

3

des Budapester Vertrags mit der Nummer DSM 4913 hinterlegt.

Es ist bekannt, daß die für Streptomyces lividans entwickelten Protoplastierungs- und Transformationsbedingungen nicht bei allen Streptomyceten erfolgreich angewandt werden können. Im Falle des Wildtyps, S. coelicolor Müller DSM 3030, können mit der für S. lividans beschriebenen Methode (C. J. Thompson et al. (1982) J. Bacteriol. 151, 668-677) ausreichend Transformanden isoliert werden. Dagegen können nach dieser Methode keine Transformanden der Lysozym defizienten Mutante S. coelicolor Müller DSM 4913 erhalten werden. Für die Mutante DSM 4913 können allerdings mit nachfolgend beschriebenen Bedingungen, die Transformationen von Plasmid-DNA in guten Ausbeuten durchgeführt werden.

Die Mutante DSM 4913 wird in Caso-Medium, bestehend aus ca. 17 g/l Pepton aus Casein, ca. 3 g/l Pepton aus Sojamehl, ca. 2,5 g/l Glukose, ca. 5 g/l Natriumchlorid, und ca. 2,5 g/l Di-kaliumhydrogenphosphat vorkultiviert, in CaSo-Medium, das mit ca. 5 g/l Glycin angereichert wurde, überimpft und etwa 45 Stunden bei 30° C geschüttelt, bis die stationäre Wachstumsphase erreicht ist. Die optimale Wachstumszeit und -temperatur kann für jede Mutante durch Bestimmung der jeweils erhaltenen Ausbeute an Transformanden, leicht ermittelt werden.

Die Protoplastierung wird mit 0,25 mg/ml bis 1,0 mg/ml Hühnerei-Lysozym, bevorzugt 1 mg/ml in P-Puffer [Hopwood, D. et al. "Genetic Manipulation of Streptomyces: A Laboratory Manual", The John Innes Foundation, Norwich, England, S. 245 (1985)] über 30 bis 60 Minuten, bevorzugt 40 Minuten, bei Raumtemperatur unter langsamem Schütteln durchgeführt. Für die Transformation werden Thiostrepton-Resistenz kodierende Plasmid-DNA und T-Puffer (Hopwood et al. s. o., S. 246) mit den Protoplasten vermischt und anschließend in einem modifizierten R3-Weichagar (Shirahama et al., 1981, Agric. Biol. Chem. 45, 1271-1273) eingebettet, der Agar statt niederschmelzender Agarose enthält. Die eingebetteten Protoplasten werden auf Regenerationsmedium, bevorzugt R2YE-Agarplatten (Hopwood et al., s.o., S. 236), verteilt und 2 bis 6 Stunden, bevorzugt 4 Stunden, ohne Deckel in der Mitte einer Sterilwerkbank getrocknet. Nach 20 bis 26 Stunden, bevorzugt 24 Stunden, Bebrütung bei 30° C erfolgt eine Überschichtung mit 200 μg/ml Thiostrepton enthaltendem NB-Weichagar (ca. 3 g/l Bacto-Rinderextrakt, ca. 5 g/l Bacto-Pepton, ca. 6 g/l Agar) und weitere Bebrütung für 6 Tagen bei 30° C.

Für das Anlegen einer Genbank wird Gesamt-DNA des Lysozym-Produzentenstamms, bevorzugt S. coelicolor DSM 3030, isoliert und beispielsweise mit der Restriktionsendonuklease Sau3A unvollständig gespalten. Sau3A-Fragmente der Größe 2,8 bis 15 kb werden isoliert und mit dem aus S. coelicolor DSM 3030 isolierten, linearisierten und dephosphorylierten Vektor pEB15 verknüpft, der sich von dem aus S. venezuelae DSM 40755 isolierten Plasmid pSVH1 (EP 0070522) ableitet. Die Verdauung von pEB15 kann beispielsweise mit dem Restriktionsenzym BglII geschehen.

Alternativ wird Gesamt-DNA von S. coelicolor DSM 3030 oder einem anderen Produzentenstamm dieses Streptomyceten mit z.B. dem Restriktionsenzym SmaI vollständig gespalten. Durch DNA-DNA-Hybridisierung mit der für das Lysozymgen geeigneten obengenannten Oligonukleotidsonde kann ein ca. 3,3 kb großer SmaI-Fragment der Gesamt-DNA von S. coelicolor DSM 3030 identifiziert werden. Anschließend wird das identifizierte Fragment mit linearisiertem, dephosphoryliertem Vektor pGM4 (EP 0257417) verknüpft, dessen Enden durch Auffüllen mit E. coli DNA-Polymerase I (Klenow Fragment) zuvor stumpfendig gemacht wurden. Die Spaltung von pGM4 kann mit dem Restriktionsenzym BamHI geschehen.

Nach Behandlung mit T4-DNA Ligase werden die DNA-Gemische in Protoplasten der Lysozym-defizienten Mutante DSM 4913 transformiert. Die erhaltenen Klone werden 5 bis 7 Tage bei 30° C auf Agarblöcken mit SM-Medium bestehend aus ca. 20 g/l Sojamehl, ca. 20 g/l Mannit und ca. 18 g/l Agar, (pH 7,5), welches mit 30 μg/ml Thiostrepton versetzt wurde, bebrütet. Anschließend werden Lysozym bildende Klone auf Lysozymtestagarplatten mit hitzeinaktivierten Micrococcus luteus Zellen durch die Ausbildung eines trüben Lysehofes identifiziert.

Die Isolierung der Plasmide pCel1 und pCel2 aus Lysozym produzierenden Klonen und erneute Transformation in die Lysozym defiziente Mutante DSM 4913 führt in allen untersuchten Fällen zu Lysozym bildenden Transformanden. Dagegen ist nach Übertragung der Plasmide pCel1 und pCel2 in S. lividans keine durch Agardiffusionstest oder photometrische Bestimmung mit Micrococcus luteus nachweisbare Lysozymproduktion feststellbar.

Transformation der Plasmide pCel1 oder pCel2 in S. coelicolor DSM 3030 oder in davon abgeleitete Lysozym überproduzierende Mutanten oder Varianten führt dagegen zu einer 2-3-fach erhöhten Lysozymausbeute im Vergleich zu den Ausgangsstämmen. Das Plasmid pCel2 erweist sich überraschenderweise als sehr stabil in S. coelicolor DSM 3030.

Durch Klonierung des 1,2 kb AvaI-Fragments von pCel2 in den mit BglII gespaltenem Vektor pEB15 entsteht das Plasmid pCel3, welches eine dem Lysozymgen zusätzlich vorangeschaltete Promotorregion des Tyrosinasegens von S. antibioticus [Bernan et al. Gene 37, 101-110 (1985)] aufweist. Im Fall von pCel3 wird eine 3- bis 5-fache Erhöhung der Lysozymproduktion von S. coelicolor DSM 3030 erreicht.

Durch diese Erfindung wird es möglich, das bakterielle Lysozym in großen Mengen wirtschaftlich herzustellen und damit seinen Einsatz als Konservierungsstoff in Lebensmitteln oder auch für medizinische Anwendungen zu gewährleisten. Gerade für den Einsatz in Lebensmitteln zeichnet sich das Enzym durch sein pH-Optimum im leicht sauren Bereich besonders aus.

**Beispiel 1**

Spaltung von DNA mit Restriktionsendonukleasen

Einzelverdauungen mit den entsprechenden Enzymen werden gängigerweise in einem Gesamtvolumen von 10-30 μl bei der für das jeweilige Enzym optimalen Reaktionstemperatur, meist 37° C, durchgeführt. Als Puffer können die von Maniatis et al. beschriebenen Niedrig-, Mittel- und Hochsalzpuffer verwendet werden (Maniatis et al.: Molecular Cloning, A Laboratory Manual, Cold Spring Harbor, S. 104ff, 1982) oder die vom jeweiligen Hersteller speziell empfohlenen Puffer Anwendung finden. Pro μg DNA im Ansatz werden rund 1-3 U Enzym eingesetzt. Die Inkubationsdauer kann abhängig von der Hitzelabilität des eingesetzten Enzyms eine bis mehrere Stunden betragen. Bei Doppelverdauungen mit zwei unterschiedlichen Enzymen können die beiden Enzyme meist gleichzeitig dem Ansatz zugegeben werden. Unterscheiden sich die Enzyme in ihren Salzansprüchen stark, so wird zunächst mit dem die geringere Salzkonzentration benötigenden Enzym verdaut und nach Erhöhung der Salzkonzentration das zweite Enzym zugegeben. Die Vollständigkeit der Verdauung kann durch die Gelelektrophorese eines Aliquots des Reaktionsgemisches überprüft werden.

**Beispiel 2**

Phosphatase-Reaktion

Die linearisierte, in TE-Puffer (10 mM Tris-HCl, 1 mM EDTA, pH 8,0) gelöste Vektor-DNA mit einem überhängenden 5'-Ende wird mit 5 μl 20x RAP Puffer (800 mM Tris-HCl, 100 mM $MgCl_2$, 2 mM $ZnCl_2$, pH 8,0) und Wasser auf ein Volumen von 100 μl gebracht und mit rund 30 U Calf Intestinal Phosphatase (CIP) (Boehringer, Mannheim) versetzt. Nach Inkubation bei 37° C für 2-3 h wird nach Zugabe von 5 μl 10% SDS für 15 min auf 68° C erhitzt. Nach dreimaligem Phenolisieren erfolgt Ethanolfällung, Waschen mit 70%igem Ethanol und erneute Aufnahme in TE-Puffer.
Alternativ kann dem Ansatz direkt im Anschluß an eine Restriktionsverdauung 1 μl CIP zugegeben und für weitere 20 min bei 37° C inkubiert werden. Dann erfolgt Zugabe von 2 μl 0,5 M EDTA und Erhitzen des Ansatzes auf 65° C für eine Stunde. Anschließend wird phenolisiert und gefällt wie oben beschrieben.

**Beispiel 3**

Ligase-Reaktion

Das Ligieren von isolierten DNA-Fragmenten und Plasmid-oder Cosmid-Vektoren erfolgt unter den von Maniatis et al. (Maniatis et al., s.o. S. 286ff; S.474) empfohlenen Bedingungen. Die Reaktionen werden in Volumina von 10-20 μl und DNA-Konzentrationen von 100-400 μg/ml durchgeführt. Besonders vorteilhaft werden die zu ligierenden DNAs gemeinsam gefällt und in einem berechneten Volumen Ligase-Puffer resuspendiert. Bei Ligationen mit Plasmiden liegt das Verhältnis von Vektorplasmid zu Insert meist zwischen 1 : 1 und 1:5; bei Ligationen mit Cosmiden umgekehrt zwischen 5:1 und 10:1. Als Enzym findet die T4-DNA-Ligase (Boehringer, Mannheim; New England Biolabs) in Konzentrationen zwischen 100 und 400 U pro Ansatz Anwendung. Die Inkubation erfolgt bei 14-18° C für mindestens 12 Stunden. Zur Überprüfung der Reaktion wird nach der Ligation ein Aliquot des Reaktionsgemisches, z.B. 3 μl bei einem 20 μl Ansatz, aus dem Reaktionsgefäß entnommen und auf einem 0,8%igen Agarose-Gel mit einer Probe verglichen, die vor zugabe der T4-DNA-Ligase dem Reaktionsgemisch entnommen und bei 4° C aufbewahrt wurde.

5

**Beispiel 4**

Herstellung von Packextrakten

Die für in vitro Verpackung von DNA in die Köpfe des Phagen lambda notwendigen Packextrakte werden nach der Methode von Hohn hergestellt (Hohn, B., in Wu, R., editor: Recombinant DNA, Methods in Enzymology, Vol 68, Academic Press, New York, S. 299-309, 1979).

Die E.coli Stämme BHB2688 (Freeze Thaw Lysate, FTL) und BHB2690 (Sonic Extract, SE) werden aus Glycerinkulturen in L-Broth Medium (10 g Bacto Trypton, 5 g Hefe Extrakt, 5 g NaCl pro Liter, pH 7,5) angeimpft (50 μl Glycerinkultur auf 5 ml Medium). Aus den bei 32° C gewachsenen Kulturen wird auf L-Broth Platten ausgestrichen und diese wieder bei 32° C bebrütet. Von den bewachsenen Platten werden jeweils 5 Kolonien gepickt und auf 2 L-Broth Platten übertragen, von denen eine bei 32° C und die andere bei 42° C bebrütet wird. Für die weiteren Arbeiten werden nur Einzelkolonien verwendet, die bei 32° C, nicht aber bei 42° C anwachsen konnten. Jeweils eine der Kolonien, die bei 42° C kein Wachstum zeigt, wird wiederum auf L-Broth Platten ausgestrichen und über Nacht bei 32° C bebrütet. Von diesen Platten wird für das Herstellen der Packextrakte jeweils eine kleine Kolonie ausgewählt und in 10 ml L-Broth Medium kultiviert. Die Kulturen werden über Nacht bei 32° C bebrütet und anderntags zur Beimpfung der Hauptkulturen verwendet. Für die Herstellung des FTL-Extraktes werden 800 ml frisches L-Broth Medium 10 %ig mit einer Vorkultur von BHB2688 beimpft und bis zu einer OD600 von 0,45-0,50 bei 32° C und 220 U/min auf einem Schüttler angezüchtet. Dann wird die Kultur mit einer Bunsenflamme unter Schwenken schnell auf 42-43° C erwärmt. Die Temperaturmessung erfolgt unsteril mit einem direkt in die Suspension eintauchenden Thermometer. Dann wird in einem Schwenkwasserbad 15 min unter leichtem Schütteln bei 42-44° C inkubiert, anschließend wird 2,5 h bei 37° C kräftig geschüttelt. Nach dem Abkühlen der Suspension auf 10° C in Eiswasser werden je 200 ml in der Sorvall RC-5 bei 6000 U/min, 4° C, für 10 min zentrifugiert. Jedes Pellet wird mit 0,3 ml einer kalten Saccharose-Lösung (10 % Sucrose in 50 mM Tris, pH 7,4) unter Vermeidung von Luftblasen vermischt, in 10 ml Zentrifugenröhrchen überführt und mit 30 μl einer frischen Lysozym-Lösung (2 mg/ml Wasser) verrührt. Die Mischung wird in Trockeneis eingefroren. Nach frühesten 30 min können die Röhrchen bei Raumtemperatur langsam aufgetaut werden. Nach vollständigem Auftauen wird dem Röhrcheninhalt jeweils 250 μl eiskalter M1-Puffer (410 μl H₂O; 1 μl β-Mercaptoethanol; 6 μl 0,5 M Tris-HCl pH 7,4; 300 μl 50 mM Spermidine; 9 μl 1 M MgCl₂; 75 μl 0,1 M ATP) zugefügt, vorsichtig gemischt und bei 35.000 U/min, 2° C, 25 min zentrifugiert (Beckman Ultrazentrifuge L8-M60). Anschließend werden die Überstände vereinigt, in 25 μl Aliquots abgefüllt in Trockeneis eingefroren und bis zur Verwendung bei -86° C aufbewahrt.

Für die Herstellung des SE-Packextraktes werden 400 ml L-Broth Medium mit 4 ml einer Übernachtkultur des Stammes BHB2090 beimpft und dann die für den Stamm BHB2688 beschriebenen Verfahrensschritte bis zur Zentrifugation in der Sorvall-Zentrifuge exakt gleich durchgeführt. Das in 2 Zentrifugenbechern anfallende Pellet des Stammes BHB2690 wird jeweils in 3 ml kaltem Puffer A (20 mM Tris-HCl (pH 8,0); 3 mM MgCl₂; 10 mM β-Mercaptoethanol; 1 mM EDTA) unter Vermeidung von Luftblasen verrührt und die Suspensionen in einem klaren Plastikgefäß vereinigt. Das Plastikröhrchen wird in eine Salz/Eis/Wasser Mischung gestellt und bei maximaler Leistung in Pulsen von 5 sec am Ultraschallgerät (Branson Sonifier) beschallt. Zwischen den Pulsen wird mit einer Pasteurpipette die Viskosität der Lösung überprüft. Die starke Zunahme der Viskosität in der Lösung nach den ersten Pulsen (Austritt hochmolekularer DNA aus den Zellen) wird mit zunehmender Pulszahl von einer Viskositätsabnahme gefolgt (Zerbrechen der DNA in niedermolekulare Fragmente). Zwischen den Pulsen wird die Suspension immer wieder soweit abgekühlt, daß die Temperatur innerhalb der Suspension nie 20° C übersteigt. Bis zur Dünnflüssigkeit der beschallten Suspension sind bis zu 10 Pulse notwendig. Anschließend an das Beschallen wird die Suspension für 10 min bei 6.000 U/min und 4° C zentrifugiert (Sorvall Kühlzentrifuge RC-5, SS-34 Rotor) und der Überstand vom kleinen Pellet abgezogen. Pro 200 μl Überstand wurden 0,6 ml kalter M1-Puffer (s.o.) zugegeben, vermischt und jeweils 20 μl Aliquots in 0,5 ml Eppendorf Reaktionsgefäße abgefüllt, welche auf Trockeneis vorgekühlt wurden. Die Aliquots werden bei -86° C aufbewahrt.

Alternativ können vorbereitete Packextrakte auch von verschiedenen Herstellern fertig bezogen werden, z.B. von den Firmen Boehringer Mannheim oder Amersham Buchler, Braunschweig.

**Beispiel 5**

Verpackung in lambda-Phagen

3 μl der in Beispiel 3 beschriebenen Ligase-Mischungen werden im Eisbad mit 9 μl eines SE-Extraktes (Beispiel 4) verrührt und nach 2 min werden 10 μl eines FTL-Extraktes (Beispiel 4) zugefügt. Dann wird 60 min bei Raumtemperatur inkubiert. Anschließend werden 500 μl SM-Puffer (100 mM NaCl, 10 mM MgS04, 50 mM Tris-HCl pH 7,5, 0,01% Gelatine) zugegeben. Diese Mischung kann direkt in eine Transduktions-Reaktion eingesetzt werden.

**Beispiel 6**

Transduktion von E.coli ED8767

5 ml L-Broth Medium, das zusätzlich noch 0,4% Maltose und 10 mM $MgSO_4$ enthält, wird mit 50 μl einer gut gewachsenen Flüssigkultur des E.coli Stamms ED8767 beimpft. Die Bakterien werden rund 12 Stunden bei 37° C geschüttelt und nach Erreichen der frühen stationären Phase in der Tischzentrifuge geerntet. Der Niederschlag wird mit 0,5 ml Puffer (10 mM $MgSO_4$, 0,4% Maltose) gewaschen und in 0,25 ml desselben Puffers resuspendiert. 100 μl dieser konzentrierten Bakteriensuspension werden mit 10 μl der unter Beispiel 5 erhaltenen Cosmid-Phagen versetzt und 15 bis 30 min bei 37° C inkubiert. Dann werden 0,5 ml L-Broth Medium zugegeben und die Mischung nochmals 30-60 min bei 37° C inkubiert. Anschließend werden 100 μl Aliquots des Ansatzes auf L-Broth Platten ausplattiert, die 50 μg/ml Ampicillin enthalten. Die Platten werden bis zum Erhalt gut gewachsener Kolonien bei 37° C inkubiert.

**Beispiel 7**

Partielle Verdauung von DNA

Die partiellen Verdauung von Gesamt-DNA aus Streptomyceten erfolgt nach dem in der Zeitschrift "focus" (Bethesda Research Laboratories, Vol.7, Nr.2, 1985, Seite 3) beschriebenen Verfahren. Aus dem Streptomycetenstamm S.coelicolor DSM 3030 isolierte Gesamt-DNA wird mit dem Restriktionsenzym SalI in Fragmente von 30-40 kb gespalten. Als besonders geeignet erweist sich ein Ansatz im Gesamtvolumen von 100 μl, in dem 50 μl DNA-Lösung, 40 μl TE-Puffer (10 mM Tris-HCl, 1 mM EDTA, pH 8,0), 10 μl 10x high salt Puffer sowie 10 U an SalI enthalten sind. Der Ansatz wird 10 min bei 37° C inkubiert, dann sofort auf Eis überführt und die DNA nach Extraktion mit Phenol/Chloroform mit Ethanol gefällt und nach Waschen mit 70% Ethanol in 50 μl TE-Puffer resuspendiert. Das Enzym SalI spaltet bei diesem Vorgehen nicht jede mögliche Schnittstelle.

**Beispiel 8**

Anlage einer Cosmid-Genbank von S.coelicolor DSM 3030 DNA in E.coli

Aus dem Streptomyceten-Stamm S.coelicolor DSM 3030 wird nach dem in D.A. Hopwood et al., s.o., S. 79 beschriebenen Verfahren Gesamt-DNA isoliert und wie in Beispiel 7 beschrieben mit dem Restriktionsenzym SalI partiell so verdaut, daß hauptsächlich DNA-Fragmente einer Größe von 30-40 kb gebildet werden. Ein Aliquot der verdauten Streptomyceten-DNA wird mit dem Cosmid-Vektor pHC79, der durch Verdauung mit dem Restriktionsenzym SalI vollständig gespalten und anschließend dephosphoryliert wird (s. Beispiel 2) wie unter Beispiel 3 beschrieben zusammenligiert. 3 μl eines 20 μl Ligase-Ansatzes werden in die Köpfe von lambda-Phagen verpackt (Beispiele 4-6). Mit den resultierenden rekombinanten Cosmid-Phagen wird der E.coli Stamm ED8767 infiziert. Die Selektion rekombinanter Bakterienkolonien erfolgt auf L-Broth Platten, denen 50 μg/ml an Ampicillin (Amp) zugesetzt wurde. Rund 2.000 Kolonien werden gepickt und auf frischen L-Broth/Amp Platten in ein Raster übertragen, so daß die einzelnen Klone leicht wiederzufinden sind. Von jeder Platte wird ein Doppel angelegt. Zum Test auf die Vollständigkeit der Genbank werden Einzelkolonien in Minilyse genommen und die isolierten Cosmid-DNAs mit dem Enzym

7

SalI vollständig gespalten. Das erhaltene Fragmentmuster zeigt, daß in allen Klonen Cosmid-DNA der Größe 30-40 kb enthalten ist.

Um in der Genbank nach dem Lysozym-Gen zu suchen, werden jeweils 10 der ins Raster gepickten Kolonien gemeinsam in 5 ml L-Broth Medium mit 50 μg/ml Ampicillin eingeimpft und über Nacht bei 37° C geschüttelt. Dann werden von den jeweiligen Suspensionen Minilysen gemacht und das erhaltene Gemisch von jeweils 10 Cosmid-DNAs mit dem Restriktionsenzym SalI vollständig gespalten. Die Fragmente werden auf 0,8%igen Agarose-Gelen aufgetrennt und mittels Southern Blotting auf Nylon-Filter übertragen. Zur Kontrolle wird auf dasselbe Gel auch SalI verdaute Gesamt-DNA des Streptomyceten S.coelicolor DSM 3030 aufgetragen. Nach Fixierung der DNA auf den Filtern kann diese direkt in die anschließenden Hybridisierungsreaktionen eingesetzt werden.

Diese Filter werden, wie in Beispiel 13 beschrieben, mit der folgenden radioaktiv markierten Oligonukleotidsonde hybridisiert:

5'-TTC GC (G/C)TAC ATC AAG GC(G/C) AC(G/C) GAG GG(G/C) AC(G/C) AAC TAC AA-3'.

Die Filter werden bei 55-60° C hybridisiert und anschließend bei 60-65° C gewaschen.

Die Cosmid-DNAs der rund 2.000 erhaltenen Cosmid-Klone werden in 10er Pools mit dem radioaktiv markierten Oligonukleotidgemisch obiger Sequenz umgesetzt. Nach Autoradiographie konnten insgesamt 11 Pools mit jeweils 10 Klonen ermittelt werden, die ein Signal liefern, das in seiner Position auf dem Filter mit dem Signal aus der S. coelicolor DSM 3030 Gesamt-DNA identisch ist. Die jeweiligen Klone aus den 10er Pools wurden einzeln angeimpft und die Cosmid-DNAs isoliert. Durch "dot spot" Hybridisierung gegen das schon zuvor verwendete Oligonukleotid werden dann die Klone identifiziert, die in den 10er Pools jeweils für das Auftreten des positiven Signals verantwortlich sind.

**Beispiel 9**

Subklonierung von DNA-Fragmenten aus Cosmid-Klonen

Aus einem der positiven Cosmid-Klone wird die Cosmid-DNA über einen CsCl/EtBr-Gradienten gereinigt und die isolierte DNA mit dem Restriktionsenzym SalI vollständig verdaut. Die resultierenden DNA-Fragmente werden mit dem Vektor pSVB26 zusammenligiert, der ebenfalls SalI verdaut und anschließend noch mit alkalischer Phosphatase (Calf Intestinal Phosphatase, Boehringer Mannheim, s. Beispiel 2), behandelt wurde.

pSVB 26 ist fast identisch mit dem bekannten Vektor pUC 8 [Vieira and Messing, Gene 19 (1982) 259-68] und unterscheidet sich lediglich durch die Orientierung und Art der Restriktionsschnittstellen im Polylinker. Für die nachfolgend beschriebene Klonierung kann ebenso pUC8 verwendet werden.

Nach Transformation von kompetenten Zellen des E.coli Stammes HB101 werden Plasmid-DNA enthaltende Klone auf L-Broth Medium selektioniert, das 50 μg/ml Ampicillin enthielt. Nach Anwachsen der Kolonien wird durch Koloniehybridisierung (Beispiel 15) untersucht, welcher Klon das interessierende Fragment enthält. Aus den positiv reagierenden Klonen wird durch Minilyse Plasmid-DNA isoliert und durch SalI-Verdauung überprüft, ob das gesuchte SalI-Fragment in den Klonen enthalten ist. Durch Hybridisierung gegen das zum Screenen verwendete Oligonukleotid werden die SalI-Fragmente endgültig identifiziert.

Durch eine ähnliche Vorgehensweise können auch andere, mit der Oligonukleotid Sonde positiv reagierende Restriktionsfragmente aus dem Cosmid-Klon in den Vektor pSVB26 subkloniert werden. Beispielsweise wurde ein rund 1,2 kb großes AvaI-Fragment aus dem Cosmid Klon identifiziert und nach Auffüllen der überhängenden Enden mittels Klenow Polymerase und Isolierung aus einem Agarose-Gel in die SmaI-Schnittstelle von pSVB26 kloniert. Ebenso werden durch den Einsatz unterschiedlicher Oligonukleotide 2 verschiedene BssHII-Fragmente identifiziert und nach Auffüllen der Enden und Isolierung aus einem Agarose-Gel in die einzige SmaI-Schnittstelle von pSVB26 kloniert. Verschiedene Restriktionsfragmente liegen damit in den Vektor pSVB26 kloniert vor.

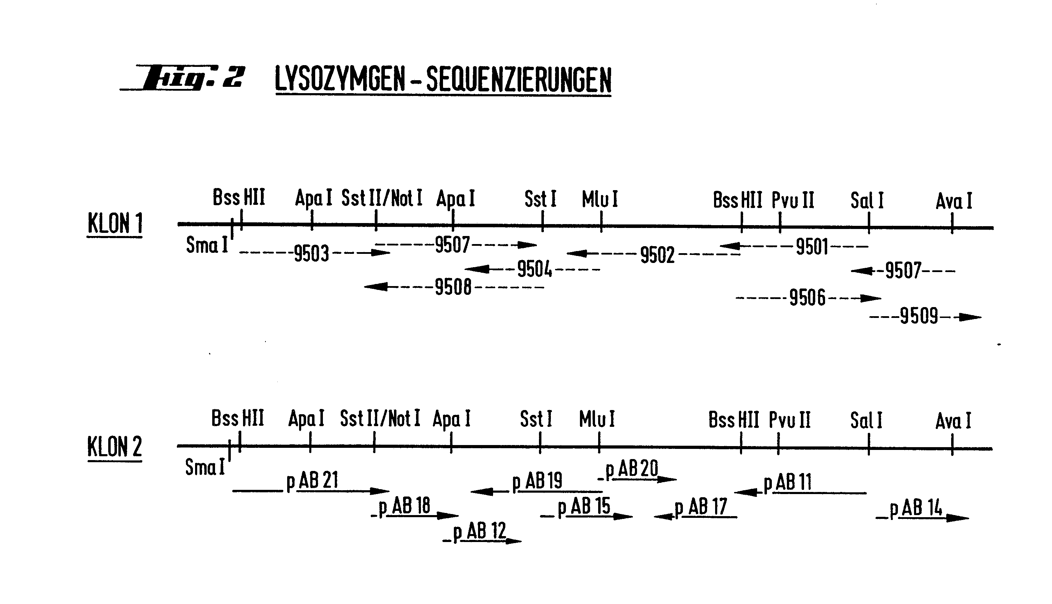

Durch Restriktionskartierung dieser Fragmente wird die in Figur 1 dargestellte Restriktionskarte erhalten, die das Lysozym-Gen sowie umgebender Bereiche beinhaltet. Die in diesem Bereich enthaltenen singulären Restriktionsschnittstellen ermöglichen es, durch Doppelverdauung mit dem entsprechenden Enzym sowie einem Enzym, das eine der Restriktionsschnittstellen im Polylinker von pSVB26 erkennt, gezielte Deletionen vorzunehmen und diese für die nachfolgende Sequenzierung mit zu verwenden. Zur Sequenzierung des SalI Fragments nach der Sanger-Methode werden die üblichen pUC-primer verwendet, da sich pSVB26 nur durch die Polylinker-Sequenz von den pUC-Vektoren unterscheidet. Außerdem werden die zum Screenen eingesetzten Oligonukleotide als Primer verwendet. Die verschiedenen in die Sequenzierung eingesetzten Konstruktionen sind in Figur 2 nochmals detailliert erläutert. Die Sequenz des in Figur 2 angegebenen 1,2 kb SmaI/AvaI-Fragmentes ist in Tabelle 1 dargestellt, die des Lysozym-Strukturgens in Tabelle 2.

Tabelle 1:

```
                                                        SmaI
                                                   CCCG GGGTGCGGACCG GCAGGTCA
GGCGCGCCGCCATTGC TCTAGACCGGCG GAGCCGACAGAT GGGTCTTTACGG GGGTCTAC
GCGCGTGCATTACTTG TGTCGTGCTCAT GGCGACCGCCCT TTCCGGGCCCCT TCGCCGGG
GCAAGCACCGGTCCTT CGCGCGTTCCAC GGCTACATCCCC CACTCGTGCCTG GAGGCAGT
CATGCCCGCGTACAGC TCTCTCGCACGC CGCGGCCGCAGA CCCGCGGTCGTC CTCCTCGG
CGGTCTCGTCAGCGCC TCCCTGGCGCTC ACCCTGGCGCCC ACCGCCGCCGCC GCGCCCCT
CGCGCCCCCGCCCGGC AAGGACGTCGGG CCCGGCGAGGCG TACATGGGTGTC GGCACCCG
CATCGAGCAGGGGCTC GGCGCCGGCCCC GACGAGCGCACC ATCGGCCCGGCC
```


        Asp ThrSerGlyVal GlnGlyIleAsp ValSerHisTrp GlnGlySerIle
        GAC ACCAGCGGTGTC CAGGGGATCGAC GTGTCGCACTGG CAGGGCTCCATC

AsnTrpSerSer ValLysSerAla GlyMetSerPhe AlaTyrIleLys AlaThrGluGly
AACTGGAGCTCG GTGAAGTCGGCC GGGATGTCCTTC GCCTACATCAAG GCGACCGAGGGC

ThrAsnTyrLys AspAspArgPhe SerAlaAsnTyr ThrAsnAlaTyr AsnAlaGlyIle
ACCAACTACAAG GACGACCGGTTC AGCGCGAACTAC ACCAACGCGTAC AACGCGGGGATC

IleArgGlyAla TyrHisPheAla ArgProAsnAla SerSerGlyThr AlaGlnAlaAsp
ATCCGGGGCGCC TACCACTTCGCC CGCCCGAACGCC TCCAGCGGCACG GCGCAGGCCGAC

TyrPheAlaSer AsnGlyGlyGly TrpSerArgAsp AsnArgThrLeu ProGlyValLeu
TACTTCGCCAGC AACGGCGGCGGC TGGTCCCGCGAC AACCGGACCCTG CCGGGCGTCCTG

AspIleGluHis AsnProSerGly AlaMetCysTyr GlyLeuSerThr ThrGlnMetArg
GACATCGAGCAC AACCCCTCCGGC GCCATGTGCTAC GGGCTCTCCACC ACGCAGATGCGC

ThrTrpIleAsn AspPheHisAla ArgTyrLysAla ArgThrThrArg AspValValIle
ACCTGGATCAAC GACTTCCACGCC CGGTACAAGGCG CGCACCACCCGC GACGTCGTCATC

TyrThrThrAla SerTrpTrpAsn ThrCysThrGly SerTrpAsnGly MetAlaAlaLys
TACACCACGGCG AGCTGGTGGAAC ACCTGCACCGGC AGCTGGAACGGC ATGGCGGCCAAG

SerProPheTrp ValAlaHisTrp GlyValSerAla ProThrValPro SerGlyPhePro
TCCCCGTTCTGG GTGGCCCACTGG GGCGTGAGCGCC CCGACGGTGCCG AGCGGCTTCCCG

ThrTrpThrPhe TrpGlnTyrSer AlaThrGlyArg ValGlyGlyVal SerGlyAspVal
ACCTGGACGTTC TGGCAGTACTCG GCGACCGGCCGG GTCGGCGGCGTC AGCGGGGACGTC

AspArgAsnLys PheAsnGlySer AlaAlaArgLeu LeuAlaLeuAla AsnAsnThrAla
GACCGCAACAAG TTCAACGGCTCC GCCGCCCGTCTG CTGGCCCTGGCC AACAACACGGCG

Stp
TGA

GACGGCCGGAGG GCCGGGGGCACG GCACGCACGCCC TGCTCCCGGCCC TCCCCCGCGCCC
GGCGCGGCTACC GCATCCGCCCGA G
                          AvaI

Tabelle 2:

```
       Asp ThrSerGlyVal GlnGlyIleAsp ValSerHisTrp GlnGlySerIle
       GAC ACCAGCGGTGTC CAGGGGATCGAC GTGTCGCACTGG CAGGGCTCCATC

AsnTrpSerSer VallysSerAla GlyMetSerPhe AlaTyrIleLys AlaThrGluGly
AACTGGAGCTCG GTGAAGTCGGCC GGGATGTCCTTC GCCTACATCAAG GCGACCGAGGGC

ThrAsnTyrLys AspAspArgPhe SerAlaAsnTyr ThrAsnAlaTyr AsnAlaGlyIle
ACCAACTACAAG GACGACCGGTTC AGCGCGAACTAC ACCAACGCGTAC AACGCGGGGATC

IleArgGlyAla TyrHisPheAla ArgProAsnAla SerSerGlyThr AlaGlnAlaAsp
ATCCGGGGCGCC TACCACTTCGCC CGCCCGAACGCC TCCAGCGGCACG GCGCAGGCCGAC

TyrPheAlaSer AsnGlyGlyGly TrpSerArgAsp AsnArgThrLeu ProGlyValLeu
TACTTCGCCAGC AACGGCGGCGGC TGGTCCCGCGAC AACCGGACCCTG CCGGGCGTCCTG

AspIleGluHis AsnProSerGly AlaMetCysTyr GlyLeuSerThr ThrGlnMetArg
GACATCGAGCAC AACCCCTCCGGC GCCATGTGCTAC GGGCTCTCCACC ACGCAGATGCGC

ThrTrpIleAsn AspPheHisAla ArgTyrLysAla ArgThrThrArg AspValValIle
ACCTGGATCAAC GACTTCCACGCC CGGTACAAGGCG CGCACCACCCGC GACGTCGTCATC

TyrThrThrAla SerTrpTrpAsn ThrCysThrGly SerTrpAsnGly MetAlaAlaLys
TACACCACGGCG AGCTGGTGGAAC ACCTGCACCGGC AGCTGGAACGGC ATGGCGGCCAAG

SerProPheTrp ValAlaHisTrp GlyValSerAla ProThrValPro SerGlyPhePro
TCCCCGTTCTGG GTGGCCCACTGG GGCGTGAGCGCC CCGACGGTGCCG AGCGGCTTCCCG

ThrTrpThrPhe TrpGlnTyrSer AlaThrGlyArg ValGlyGlyVal SerGlyAspVal
ACCTGGACGTTC TGGCAGTACTCG GCGACCGGCCGG GTCGGCGGCGTC AGCGGGGACGTC

AspArgAsnLys PheAsnGlySer AlaAlaArgLeu LeuAlaLeuAla AsnAsnThrAla
GACCGCAACAAG TTCAACGGCTCC GCCGCCCGTCTG CTGGCCCTGGCC AACAACACGGCG

Stp
TGA
```

**Beispiel 10**

Identifizierung hybridisierender Fragmente aus Gesamt-DNA

Isolierte Gesamt-DNA aus S.coelicolor wird mit unterschiedlichen Restriktionsenzymen, beispielsweise SalI, BssHII, SmaI, AvaI, BgIII, BamHI, EcoRI, PvuII und anderen jeweils einzeln vollständig verdaut. Die resultierenden Fragmentgemische werden auf 0,8%igen Agarose-Gelen aufgetrennt und durch Southern Blotting auf Nylon-Filter übertragen. Die Filter werden wie in Beispiel 13 angegeben behandelt und gegen radioaktiv markierte Oligonukleotide hybridisiert. Die optimale Hybridisierungstemperatur wird durch Variation der Hybridisierungs- und Waschtemperaturen zwischen 30-60°C bestimmt. Die Hybridisierung wird mittels Autoradiographie sichtbar gemacht. Auf diese Art wird für jedes Oligonukleotid(gemisch) nach der Temperatur gesucht, bei der aus der aufgetrennten Gesamt-DNA nur ein Fragment, bei möglichst hoher Hybridisierungs- und Waschtemperatur, identifiziert werden kann. Besonders gute Ergebnisse werden mit dem 38 Nukleotide langen Oligonukleotidgemisch der Sequenz 5'-TTC GC (G/C)TAC ATC AAG GC(G/C) AC(G/C) GAG GG(G/C) AC(G/C) AAC TAC AA-3' erzielt. Kürzere Oligonukleotidgemische liefern unbefriedigende Ergebnisse. Mit dem 38 Nukleotide langen Oligonukleotidgemisch werden bei Hybridisierungs- und Waschtemperaturen von 60-65°C eindeutige Signale aus der S. coelicolor DSM 3030 Gesamt-DNA erhalten. Beispielsweise werden so ein rund 3,3 kb großes SmaI-, ein rund 1,3 kb großes SalI-, ein rund 1,2 kb großes AvaI- und ein rund 0,8 kb großes BssHII-Fragment aus der Gesamt-DNA von S. coelicolor DSM

10

3030 mit diesem Oligonukeotidgemisch identifiziert. Bei Verdauungen mit EcoRI, BamHI, BglII, XhoI und anderen werden nur sehr große Fragmente gefunden.

## Beispiel 11

Klonierung eines SalI-Fragments aus S. coelicolor DSM 3030 Gesamt-DNA

Nach dem in Beispiel 10 erläuterten Verfahren konnte ein rund 1,3 kb großes SalI-Fragment identifiziert werden, das mit einem zur Suche nach dem Lysozym-Gen eingesetzten Oligonukleotidgemisch ein positives Signal liefert. Gesamt-DNA von S.coelicolor DSM 3030 wurde vollständig mit dem Restriktionsenzym SalI verdaut und Fragmente der Größe 1,1-1,5 kb nach dem in Beispiel 21 erläuterten Verfahren aus einem Agarose-Gel isoliert. Die gereinigten Fragmente werden mit dem Vektor pSVB26 zusammen ligiert, der vollständig mit SalI verdaut und anschließend mit alkalischer Phosphatase (Calf Intestinal Phosphatase, Boehringer Mannheim) behandelt wurde. Kompetente Zellen des E.coli Stammes HB101 werden mit einem Aliquot des Ligase-Ansatzes transformiert und auf L-Broth Medium mit 50 µg/ml Ampicillin plasmidhaltige Klone selektioniert. Die erhaltenen rund 1000 Kolonien werden in Raster gepickt und von jeder Platte ein Doppel angelegt. Jeweils 10 Klone werden gemeinsam in 5 ml L-Broth Medium überimpft, nach Anwachsen mittels Minilyse die Plasmid-DNAs isoliert und unverdaut auf ein Agarose-Gel aufgetragen. Nach kurzer Elektrophorese werden die DNAs durch Southern Blotting auf Nylon-Filter übertragen. Diese Filter werden sodann mit obigem Oligonukleotidgemisch unter den zuvor ermittelten optimalen Bedingungen hybridisiert. Auf diese Art wurden positive Pools gefunden.

Die in den Pools vertretenen Klone werden einzeln angeimpft und wie oben beschrieben verfahren. Dadurch kann der jeweils positiv reagierende Klon eindeutig identifiziert werden. Die aus diesen Einzel-Klonen zu isolierenden Plasmide enthielten alle dasselbe SalI-Fragment. Es wurde weiter vorgegangen wie unter Beispiel 9 beschrieben.

## Beispiel 12

DNA-Transfer nach Southern

Fragmentierte DNA wird auf 0,8%igen Agarose-Gelen in Gegenwart eines DNA-Längenstandards elektrophoretisch aufgetrennt. Nach Beendigung des Laufs wird das Gel in eine Plastikschüssel transferiert und 15-20 min mit 0,25 M Salzsäure, dann 2x30 min mit Denaturierungspuffer (0,5 M NaOH, 1,5 M NaCl) und schließlich 2x30 min mit Neutralisierungspuffer (1 M Tris-HCl (pH 8,0), 1,5 M NaCl) auf einem Taumelgerät behandelt. Es wird jeweils soviel Pufferlösung zugegeben, daß das Gel gut bedeckt ist. Anschließend wird das Gel auf 2 Lagen saugfähiges Papier (Whatman, 3 MM) gelegt und der Blot so aufgebaut, wie er in Maniatis et al. (s.o.) auf S. 385 beschrieben ist. Anstelle eines Nitrocellulose-filters wird allerdings ein Nylon-Filter (Genescreen Plus, Firma NEN) verwendet. Der Lauf wird durch Auflegen der Saugpapiere gestartet und mindestens 16 Stunden durchgeführt. Nach Beendigung des Transfers werden die Geltaschen markiert und das Nylon-Filter bei 65°C im Trockenschrank getrocknet. Anschließend wird das Filter auf einem mit 10xSSC-puffer getränkten Saugpapier (Whatman, 3 MM) eingeweicht und mit kurzwelligem UV-Licht 2 min lang bestrahlt (UV-Lampe, Desaga Minuvis, Wellenlänge 254 nm, Abstand Filter/UV-Quelle 10-12 cm). Die mit DNA beladene Seite des Filters ist der UV-Quelle dabei abgewandt. Nach der Bestrahlung wird das Filter an Luft getrocknet.

## Beispiel 13

Hybridisierung von filtergebundener DNA

Die Hybridisierung wurde im wesentlichen so durchgeführt, wie sie in dem von der Firma Gibco BRL herausgegebenen Heft "focus", Vol.9 Nr.2, 1987, auf den Seiten 1-2 beschrieben ist. Das Filter wird mindestens 2 h mit Vorhybridisierungs-Puffer bei 65°C gewaschen (5xSSC, 20 mM Natriumphosphat pH 7,0, 10xDenhardt, 7% SDS, 100 μg/ml durch 15 min Kochen denaturierte Lachssperm-DNA. Dann wird Dextran-Sulfat Lösung (50%) bis zu einer Endkonzentration von 10% zugefügt und radioaktiv markiertes Oligonukleotid bis zu einer Konzentration von 1-5 ng/ml Hybridisierungslösung zugegeben. Anschließend wird mindestens 16 h im Schüttelwasserbad unter leichtem Schütteln inkubiert. Die jeweilige Inkubationstemperatur ist vom Oligonukleotid abhängig und wird vorher optimiert. Die Filter werden in Waschpuffer I (3xSSC, 10 mM Natriumphosphat (pH 7,0), 10xDenhardts, 5% SDS) gegeben. Nach einer Stunde Schütteln bei der Hybridisierungstemperatur wird der Waschpuffer I durch Waschpuffer II ersetzt (1xSSC, 1% SDS) und dieser nach 30 min gewechselt. Nach 30 min in frischem Waschpuffer II wird das feuchte Filter in einer Kassette auf röntgenempfindlichem Film exponiert (z.B. Kodak, XS1). Die Expositionszeiten betragen bei -80 °C zwischen 4 h und 5 Tagen.

**Beispiel 14**

**Autoradiographie**

Hybridisierte Filter werden auf Whatman 3 MM Papier fixiert. Unter Verwendung geeigneter Röntgenfilme (Kodak X-Omat AR; Kodak XS1) wird in einer lichtdichten Kassette mit Verstärkerfolie bei -80 °C exponiert. Die Filme werden anschließend entwickelt.

**Beispiel 15**

Koloniehybridisierung

Ein Plaque/Colony Screen Filter (Colony/Plaque ScreenTM, NEN Research Products, Katalog Nr. NEF-978/978A) wird auf eine Agarplatte mit Kolonien aufgelegt. Der Filter wird nach 2-3 min abgezogen und mit den anhaftenden Bakterienkolonien nach oben zunächst für 5 min auf ein in 10%igem SDS getränktes Whatman 3 MM Papier gelegt. Anschließend wird das Filter für 5 min auf Whatman 3 MM Papier transferiert, das zuvor in 0,5 M NaOH, 1,5 M NaCl getränkt wurde. Ein erneuter Transfer für 5 min auf Whatman 3 MM Papier, das zuvor in 0,5 M Tris-HCl (pH 8,0), 1,5 M NaCl getränkt wurde, wird von einem letzten Transfer auf trockenes Whatman 3 MM Papier gefolgt. Nach Trocknen wird das Filter mehrere Stunden in Puffer (3xSSC, 0,1% SDS) bei 65 °C geschüttelt, um anhaftende Koloniereste von den Filtern zu entfernen.

**Beispiel 16**

Synthese von Oligonukleotiden

Am Beispiel des Oligonukleotids aus Beispiel 10 wird die Synthese der DNA-Bausteine erläutert. Für die Festphasensynthese wird das am 3′-Ende stehende Nucleosid, im vorliegenden Falle also Adenin, über die 3′-Hydroxylgruppe kovalent an einen Träger gebunden verwendet. Trägermaterial ist das mit langkettigen Aminoalkylresten funktionalisierte CPG (Controlled Pore Glass). In den folgenden Syntheseschritten wird die Basenkomponente als 5-O-Dimethoxytrilylnucleosid-3-phosphorigsäure-β-cyanoethyldialkylamid eingesetzt, wobei das Adenin als N6-Benzoylverbindung, das Cytosin als N4-Benzoylverbindung, das Guanin als N2-Isobutyrylverbindung und das Thymin ohne Schutzgruppe vorliegen.

25 mg des polymeren Trägers, der 0,2 μmol 5-O-Dimethoxytrityl-N6-Benzoyl-2-deoxyadenosin gebunden enthält, werden nacheinander mit folgenden Agenzien behandelt:

    A) Acetonitril
    B) 3% Trichloressigsäure in Dichlormethan
    C) Acetonitril
    D) 5 μmol des entsprechenden Nucleosid-3-O-phosphits und 25 μmol Tetrazol in 0,15 ml wasserfreiem Acetonitril
    E) Acetonitril

F) 20% Acetanhydrid in Tetrahydrofuran mit 40% Lutidin und 10% Dimethylaminopyridin

G) Acetonitril

H) 3% Jod in Lutidin/Wasser/Tetrahydrofuran im Volumenverhältnis 5:4:1

Unter "Phosphit" wird hierbei der 2-Desoxyribose-3-monophosphorigsäure-mono-β-cyanoethylester verstanden, wobei die dritte Valenz durch einen Diisopropylaminorest abgesättigt ist. Die Ausbeuten der einzelnen Syntheseschritte können jeweils nach der Detritylierungsreaktion B) spektrophotometrisch durch Messung der Absorption des Dimethoxytritylkations bei der Wellenlänge von 496 nm bestimmt werden. Nach abgeschlossener Synthese erfolgt die Abspaltung der Dimethoxytritylgruppe wie in A)-C) beschrieben. Durch die Behandlung mit Ammoniak wird das Oligonucleotid vom Träger gespalten und zugleich werden die β-Cyanoethylgruppen eliminiert. Eine 16-stündige Behandlung der Oligomeren mit konzentriertem Ammoniak bei 50°C spaltet die Aminoschutzgruppen der Basen quantitativ ab. Das so erhaltene Rohprodukt wird durch Polyacrylamid-Gelelektrophorese gereinigt.

**Beispiel 17**

Sequenzanalyse von Proteinen

Die Sequenzanalyse von HPLC-gereinigten Proteinen oder Proteinfragmenten wird mit einem Protein Sequenzer (Applied Biosystems, 477 A), durchgeführt.

**Beispiel 18**

Sequenzanalyse von DNA

Die Sequenzanalyse von DNA wurde nach der von Sanger et al. (Proc. Natl. Acad. Sci. USA 74, S. 5463-67, 1977) entwickelten Methode der DNA-Sequenzierung mit Didesoxynukleotiden (Kettenabbruchverfahren) an doppelsträngiger Plasmid-DNA mit den von Heinrich (Guidelines for quick and simple Plasmid-Sequencing; Boehringer Mannheim, 1986) erarbeiteten Modifikationen durchgeführt. Zur Sequenzierung aus pSVB-Vektoren können die auch für das Sequenzieren aus pUC-Vektoren eingesetzten "normalen" bzw. "reversed" primer verwendet werden. Die zum Sequenzieren eingesetzte DNA wird zweimal über CsCl/EtBr-Gradienten gereinigt.

Sequenziert wird unter Verwendung von Reagenzien, die als "kit" z.B. von der Firma Boehringer Mannheim (Klenow-Polymerase) angeboten werden.

**Beispiel 19**

Sequenzgele

Die sequenzierten Proben werden auf 6%igen Polyacrylamid-Harnstoff Gelen aufgetrennt. Um die starke Auftrennung der Banden im unteren Teil des Geles zu komprimieren und im oberen Teil eine möglichst hohe Auflösung zu erreichen, wird ein Salzgradient verwendet, mit 1 M Na-Acetat im Laufpuffer des Anoden-Reservoirs.

**Beispiel 20**

Radioaktive Markierung von Oligonukleotiden

Bei der radioaktiven Markierung von gereinigten Oligonukleotiden wurde im wesentlichen so vorgegangen, wie es in dem von der Firma Gibco BRL herausgegebenen Heft "focus", Vol. 9, Nr. 2, 1987, auf Seite 1 beschrieben ist. Gereinigtes und entsalztes Oligonukleotid, mit einer optischen Dichte von 1 bei 260 nm,

wird in 40 μl bidestilliertem Wasser aufgenommen und davon standardmäßig 1 μl in die Markierungsreaktionen eingesetzt. Von der verwendeten Radioaktivität (Y-32P ATP, 6.000 Ci/mMol, Firma New England Nuclear) werden 15-20 μl einrotiert (Speed Vac Concentrator, Firma Bachhofer) und in 7 μl bidestilliertem Wasser wieder aufgenommen. Es wird 1 μl an 10x Kinase-Puffer (0,5 M Tris-Cl (pH 7,6); 0,1 M $MgCl_2$; 50 mM DTT; 1 mM Spermidin; 1 mM EDTA) zugesetzt sowie 1 μl an resuspendiertem Oligonukleotid und 1 μl T4-Polynukleotid-Kinase (ca. 7 Units). Der Ansatz wird rund 30 min bei 37° C inkubiert und nicht inkorporiertes Y-ATP über eine Sephadex G15 Säule abgetrennt. Das radioaktiv markierte Oligonukleotid kommt hierbei als erste direkt mit dem Handzähler meßbare Fraktion deutlich vor der Hauptmenge an nicht inkorporiertem Y-ATP. Das gereinigte Oligonukleotid wird direkt in die anschließenden Hybridisierungsexperimente eingesetzt.

**Beispiel 21**

Isolierung von DNA-Fragmenten aus Agarose-Gelen

Nach Auftrennung der DNA durch Gelelektrophorese in einer horizontalen Apparatur unter Verwendung von Natrium-Acetat als Laufpuffer wird unter langwelligem UV-Licht (366 nm) durch Vergleich mit Marker-Fragmenten die Lage des interessierenden DNA-Fragments bestimmt. Ein 1-2 mm tiefer Bereich in der Breite der Bande wird aus dem Gel ausgeschnitten und nach Rücktransfer des Gels in die Apparatur mit Laufpuffer gefüllt. Die Elektrophorese wird sodann für sehr kurze Zeiträume (45-60 sec) unter normalen Bedingungen wieder gestartet, der Puffer aus der ausgeschnittenen Tasche abpipettiert und dieser Vorgang so oft wiederholt, bis die interessierende DNA-Bande vollständig aus dem Gel verschwunden ist. Die aus der Geltasche abpipettierten Puffermengen werden vereinigt, mit Phenol/Chloroform extrahiert und die eluierte DNA wie üblich nach Ethanol-Fällung in TE-Puffer resuspendiert.

**Beispiel 22**

Auswahl von Oligonukleotid-Sequenzen

Durch Sequenzierung des N-Terminus sowie von BrCN-Bruchstücken des Lysozym-Proteins werden folgende Aminosäure-Sequenzen erhalten:

N-terminale Sequenz:

```
                                                    Arg           Trp
Asp-Thr-Ser-Gly-Val-Gln-Gly-Ile-Asp-Val-Ser-His-      -Gln-Gly-Ser
                                                    Trp           Gln


   Ile
     -Ile-Asn-Trp
 · Ser
```

BrCN-Bruchstück 2

```
                                                        Asp
                                Thr-Glu-Gly-Thr-Asn-Tyr-Asn-Lys-Ile
Ser-Phe-Ala-Tyr-Ile-Lys-Ala-
                                Ile-Ala-Gly-Tyr-Asp-Tyr-Phe- X -Asp
                                                    Ala

         Ser     Ile
Arg-Phe-     -Ala-    -Tyr
         Val     Asn
```

BrCN-Bruchstück 3

Ala-Ala-Lys- X - Pro-Phe-Trp-Val-Ala-His-Trp-Gly-Val-Ser-Ala-Pro-

Ser-Gly-Phe-Pro-Thr

Aus den fett gedruckten Aminosäure-Sequenzen werden folgende Oligonukleotidgemische synthetisiert:

N-terminale Sequenz; GGG/C GTG/C CAA/G GGG/C ATT/C GAT/C GT

bzw.                            CAA/G GGN   ATT/C GAT/C GT
                                für die unterstrichene Sequenz

BrCN-Bruchstück 3: TTT/C TGG GTG/C GCG/C CAT/C TGG GG
bzw. die Einzel-Oligonukleotide
TTC TGG GTG GCG CAC TGG GG
TTC TGG GTC GCC CAC TGG GG
TTC TGG GTG GCC CAC TGG GG
TTC TGG GTC GCG CAC TGG GG

Mit den aufgelisteten Oligonukleotiden konnten bei der Hybridisierung gegen genomische Blots des Produzentenstammes teilweise reproduzierbare Signale erhalten werden. Insbesondere gilt dies für das 14mer aus dem N-terminalen Bereich sowie für das 20mer aus BrCN-Bruchstück 3. Die mit den entsprechenden Oligonukleotiden identifizierten Klone erwiesen sich allerdings nicht als die richtigen.

15

Die aus BrCN-Bruchstück 2 gefundene Aminosäure-Sequenz wies abhängig vom Experiment deutliche Divergenzen auf. Es kann allerdings durch Computer-Vergleich gezeigt werden, daß die oben unterstrichene Sequenz eine deutliche bessere Übereinstimmung zu einer veröffentlichten Sequenz aus dem Pilz Chalaropsis zeigt. Aus dieser Sequenz wird unter alleiniger Berücksichtigung von G und C Resten in der 3. Position der jeweiligen Codons folgendes Oligonukleotid synthetisiert:

5-TTC GC (G/C)TAC ATC AAG GC(G/C) AC(G/C) GAG GG(G/C) AC(G/C) AAC TAC AA-3.

Mit diesem 38mer werden bei Hybridisierung gegen genomische DNA des Produzentenstammes reproduzierbare Signale erhalten.

Das Oligonukleotid wurde erfolgreich zum Auffinden des Lysozymgens aus Genbanken in E.coli eingesetzt. Durch DNA-Sequenzierung des klonierten Gens konnte gezeigt werden, daß im Gegensatz zu dem durch Aminosäuresequenzierung identifizierten Asparaginrest an Position 14 des Bromcyan Bruchstücks 2 des Lysozyms ein Lysinrest vorliegt. Die jeweiligen Codons unterscheiden sich jedoch lediglich in der 3. Position, so daß dieser Fehler keine negativen Auswirkungen auf die Bindefähigkeit des eingesetzten Oligonukleotids hatte.

**Beispiel 23**

Isolierung einer Lysozym-defizienten Mutante von S. coelicolor DSM 3030 durch chemische Mutagenese.

Zur Herstellung einer Sporensuspension wird der Lysozymproduzent S. coelicolor DSM 3030 in Schrägagarröhrchen mit SM-Medium, bestehend aus 20 g/l Sojamehl, 20 g/l Mannit und 18 g/l Agar, (pH 7,5), mindestens 7 Tage bei 30° C bebrütet, bis deutliche Sporenbildung beobachtet wird. Je ein Röhrchen wird mit 5 ml einer 0,9 % (w/v) Natriumchloridlösung, die mit 0,01 % (w/v) Polyoxyethylensorbitanmonooleat (®Tween 80) versetzt war, zwanzig Sekunden im Ultraschallbad abgeschwemmt. Zellreste werden durch Filtration durch einen sterilen Membranfilter der Porengröße 5 μm abgetrennt. Die im Filtrat enthaltenen Sporen werden durch zehn Minuten Zentrifugation bei 10000 x g gesammelt. Für die anschließende chemische Mutagenese werden mindestens $10^6$ Sporen in 2 ml 0,9 % (w/v) Natriumchlorid suspendiert, mit 8 ml einer 3 mg/ml N-Methyl-N'-nitro-N-nitrosoguanidin ("MNNG")-Lösung in TM-Puffer (50 mMol/l Tris-Maleat, pH 9,0) versetzt und 60 Minuten im Dunkeln bei Raumtemperatur inkubiert. Unter diesen Bedingungen wird eine Überlebensrate der Sporen von ca. 1 % erreicht. Die Sporen werden zehn Minuten bei 10000 x g sedimentiert, dreimal mit je 10 ml 0,9 % (w/v) Natriumchlorid gewaschen, anschließend in 5 ml 0,9 % (w/v) Natriumchlorid suspendiert, in geeigneter Verdünnung auf SM-Nährbodenplatten verteilt und mindestens fünf Tage bei 30° C bebrütet. Die erhaltenen Kolonien werden auf SM-Agarblöckchen (0,5 cm Durchmesser) überimpft und in einer feuchten Kammer fünf Tage bei 30° C bebrütet. Die Blöckchen werden anschließend auf eine Platte mit Micrococcus-Testagar umgesetzt und weitere 16 Stunden bei 30° C in einer feuchten Kammer inkubiert.

Der Lysozym-Testagar wird aus 0,4 mg/ml gefriergetrockneten Zellen von Micrococcus luteus ATCC 4698 (Boehringer Mannheim) in 0,1 molarem Natriumacetatpuffer (pH 5,0) hergestellt, die durch fünf Minuten Behandlung mit dem Ultraturrax homogenisiert wurden und vor Gebrauch 30 Minuten bei 60° C und anschließend 15 Minuten bei 42° C inkubiert und mit 0,9 % Agar versetzt.

Die Wirkung extrazellulärer Proteasen wird in dem verwendeten Micrococcus-Testagar durch die Bildung eines klaren Hofes, der direkt an die Kolonie angrenzt, angezeigt. Lysozym bewirkt das Auftreten eines weiteren daran anschließenden äußeren trüben Lysehofes.

Von 10.000 untersuchten Kolonien wurde eine Lysozym-negative Mutante von S. coelicolor DSM 3030 isoliert, die keinen trüben Lysehof mehr aufwies. Diese wurde von der entsprechenden SM-Nährboden-Platte in 100 ml SM-Nährlösung, welche mit 50 mMol/l HEPES (pH 7,0) supplementiert wurde überimpft und deren Lysozymproduktion nach drei, fünf, sieben und zehn Tagen Schütteln bei 30° C nach der in DE 3440735 beschriebenen turbidimetrischen Methode mit Micrococcus luteus bestimmt. Die isolierte Mutante ist stabil, dies zeigte sich auch bei wiederholten Schüttelkulturversuchen in SM-Nährlösung sowie nach Protoplastierung und Transformation mit dem Vektor PGM4 (EP 0257417).

**Beispiel 24**

Isolierung der Lysozym defizienten Mutante von S. coelicolor DSM 4913 durch physikalische Mutagenese

16

Eine filtrierte Sporensuspension von mindestens 10$^6$ Sporen von S. coelicolor DSM 3030 in 5 ml 0,9 % (w/v) Natriumchlorid und 0,01 % (w/v) Polyoxyethylensorbitanmonooleat (®Tween 80) wurde gemäß Beispiel 23 hergestellt. Bei der anschließenden UV-Mutagenese wurde nach der in (Hopwood D.A. et al. s.o. S. 37-38) beschriebenen Methode verfahren. Dabei wurde die Bestrahlungsdauer mit UV-Licht (254 nm) so gewählt, daß eine Überlebensrate von 2 % resultierte. Die bestrahlten Sporen wurden in geeigneter Verdünnung auf SM-Nährboden-Platten (s. Beispiel 23) verteilt und mindestens fünf Tage bei 30°C bebrütet.

Die erhaltenen Kolonien werden auf SM-Agarblöckchen (0,5 cm Durchmesser) überimpft und in einer feuchten Kammer fünf Tage bei 30°C bebrütet. Die Blöckchen werden auf eine Platte mit Micrococcus-Testagar (s. Beispiel 23) umgesetzt und weitere 16 Stunden bei 30°C in einer feuchten Kammer inkubiert. Von 7000 untersuchten Kolonien wurde die Lysozym defiziente Mutante, S. coelicolor DSM 4913, isoliert, die auf Micrococcus-Testplatten keinen trüben Lysehof aufwies. Diese Mutante erwies sich auch bei wiederholten Schüttelkulturversuchen in SM-Nährlösung, die mit 50 m Mol/l Hepes (pH 7,0) versetzt war (s. Beispiel 23), sowie nach Protoplastierung und Transformation mit Vektor pGM4 als stabil.

**Beispiel 25**

Herstellung von Protoplasten und Transformation von S. coelicolor Stämmen

Die nach Beispiel 24 isolierte Lysozym-negative Mutante S. coelicolor DSM 4913 wird in 50 ml CaSo-Nährlösung (17 g/l Pepton aus Casein, 3 g/l Pepton aus Sojamehl, 2,5 g/l Glukose, 5 g/l Natriumchlorid, 2,5 g/l Di-Kaliumhydrogenphosphat) drei Tage bei 30°C geschüttelt. 5 ml dieser Kultur werden in 50 ml CaSo-Nährlösung, die zusätzlich 5 g/l Glycin enthielt, überimpft und ca. 45 Stunden bei 30°C geschüttelt, bis die stationäre Wachstumsphase erreicht wird.

Das Zellmycel wird mit einem Glas-Homogenisator fein verteilt und 10 Minuten bei 5000 x g zentrifugiert. Die Zellen werden in 25 ml P-Puffer (Hopwood D.A. et al. s.o., S. 245) gewaschen, 10 Minuten bei 5000 x g zentrifugiert und in 12,5 ml P-Puffer aufgenommen. 12,5 ml 2 mg/ml Hühnerei-Lysozym in P-Puffer werden zugesetzt und ca. 40 Minuten bei Raumtemperatur langsam geschüttelt, bis das Mycel bei mikroskopischer Betrachtung weitgehend in Protoplasten zerfallen ist. 25 ml P-Puffer werden zugegeben, und durch Watte filtriert. Das Filtrat wird 10 Minuten bei 3000 x g zentrifugiert, die sedimentierten Protoplasten in 2 ml P-Puffer resuspendiert und in 200 μl Portionen bei -80°C eingefroren.

Bei der Transformation werden 200 μl aufgetaute Protoplasten von S. coelicolor DSM 4913 mit 20 μl Thiostrepton-Resistenz kodierender Plasmid-DNA, entsprechend 1 ng bis 1 μg DNA, mit 500 μl T-Puffer (Hopwood D.A. et al. s.o. S. 246) vermischt und sofort auf sieben auf 70 % ihres Ausgangsgewichts getrocknete R2YE-Platten (Hopwood D.A. et al. s.o. S. 236) verteilt und anschließend mit 4 ml auf 42°C vorgewärmtem modifizierten R3-Weichagar überschichtet und vier Stunden ohne Deckel in der Mitte einer Sterilwerkbank (Klasse 2) getrocknet. Der modifizierte R3-Weichagar [Shirahama et al., Agric. Biol. Chem. 45, 1271-1273 (1981)] enthält 171 g/l Saccharose, 10 g/l Glukose, 4 g/l Bacto-Pepton, 4 g/l Hefeextrakt, 0,5 g/l Kaliumchlorid, 8,1 g/l Magnesiumchlorid, 2,2 g/l Calciumchlorid, 0,2 g/l Kaliumdihydrogenphosphat, 4 g/l Agar, 25 mmol/l TES-Puffer (N-tris[Hydroxymethyl]methyl-2-aminoethansulfonsäure, pH 7,2). Die beimpften und getrockneten Platten werden 24 Stunden bei 30°C bebrütet, anschließend mit 2,5 ml auf 42°C vorgewärmten NB-Weichagar (3 g/l Bacto-Rindextrakt, 5 g/l Bacto-Pepton, 6 g/l Agar), welcher mit 200 μg/ml in Dimethylsulfoxid gelösten Thiostrepton versetzt wurde, überschichtet und mindestens weitere sechs Tage bei 30°C bebrütet.

**Beispiel 26**

Isolierung von Vektorplasmiden

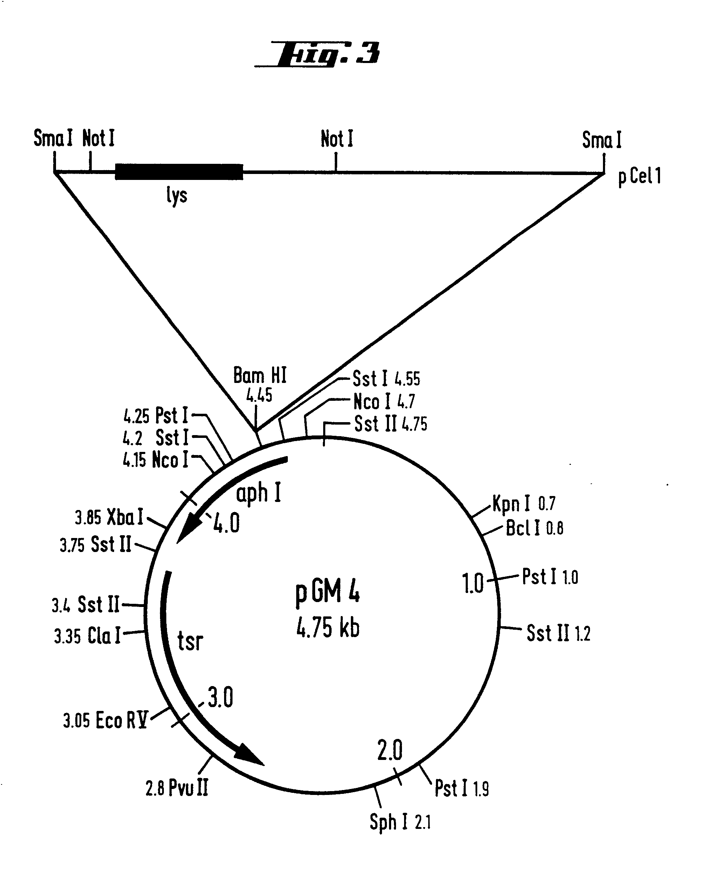

Vektor pGM4 (s. Fig. 3) wurde wie in EP 0257417 beschrieben aus dem von Streptomyces ghanaensis DSM 2932 isolierten Plasmid pSG5 hergestellt, in S. coelicolor DSM 4913 transformiert und nach der in (D.A.. Hopwood et al (s.o.), S. 85) beschriebenen Methode isoliert.

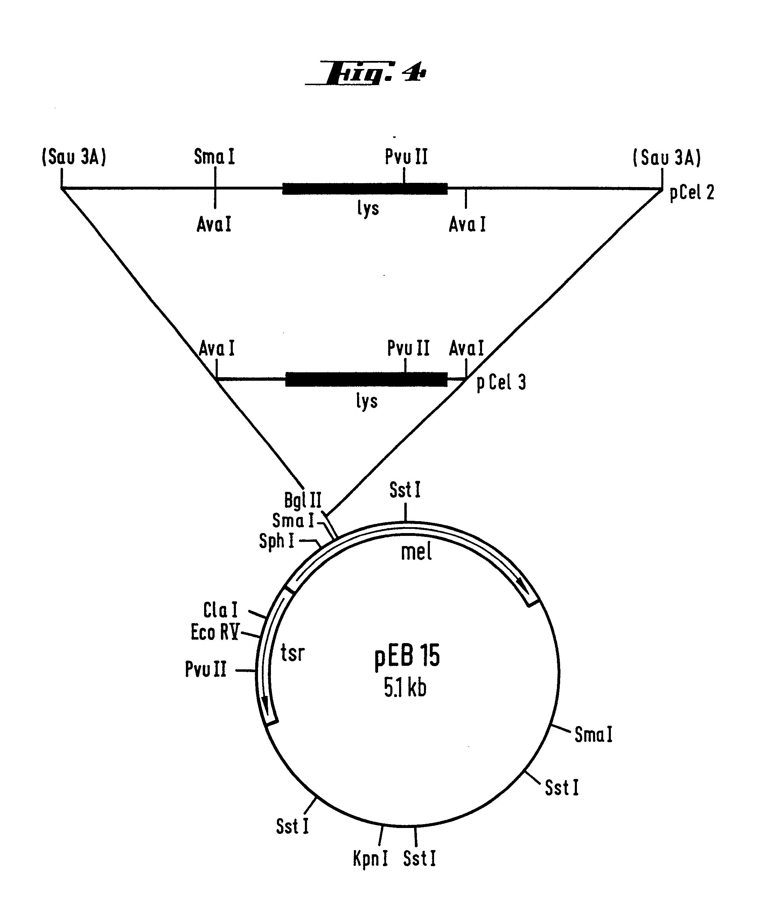

Vektor pEB15 (s. Fig. 4) leitet sich von dem aus S. venezuelae DSM 40755 gewonnenen Plasmid PSVH1 (EP 00 70 522) ab. Die beiden 2,5 kb und 3,0 kb großen BglII-Fragmente von pSVH1 werden

17

deletiert und durch ein Thiostrepton-Resistenz kodierendes 1,1 kb großes Bcl I-Fragment aus dem Vektor pSLE41 (DE 34 12 093) ersetzt, wodurch der 8,15 kb große Vektor pEB2 entsteht (Wohlleben W. et al. (1985). In: Sixth Int. Symp. on Actinomycetes Biology, Szabó G, Biró S., Goodfellow M, eds., S. 99-101. Akademiai Kiadó, Budapest).

Das 4,6 kb SstII-Fragment von pEB2 wird mit dem Neomycin-Resistenz (APH-I) kodierenden 1,0 kb SstII-Fragment des Plasmids pIJ680 (Hopwood D.A., et al. s.o. S. 298) verknüpft, wodurch das 5,7 kb Plasmid pEB3 entsteht. Durch partielle Sau3A-Verdauung von pEB3 läßt sich das Minimalreplikon von pSVH1 auf einem 2,4 kb Fragment lokalisieren.

Das 5,1 kb Vektorplasmid pEB15 (s. Fig. 4) enthält dieses Fragment sowie das Thiostrepton-Resistenz kodierende 1,1 kb BclI-Fragment von pSLE 41 (DE 34 12 093) und das benachbart lokalisierte Tyrosinase kodierende 1,6 kb BclI-Fragment des Plasmids pIJ702 [Hopwood D.A., et al. s.o. S. 292, Bernan et al. Gene 37, 101-110 (1985)]

Das Vektorplasmid pEB15 wird in S. coelicolor DSM 4913 transformiert, wodurch der Stamm S. coelicolor DSM 4914 erhalten wird. S. coelicolor DSM 4914 wurde bei der Deutschen Sammlung für Mikroorganismen und Zellkulturen hinterlegt.

Isolierung von pEB15 aus S. coelicolor DSM 4914 erfolgte ebenfalls nach der in Hopwood D.A. et al. S. 85 beschriebenen Methode.

## Beispiel 27

Klonierung von Sau3A-DNA-Fragmenten von S. coelicolor DSM 3030 in die Lysozym defiziente Mutante DSM 4913

Gesamt-DNA des Lysozymproduzenten S. coelicolor DSM 3030 wurde nach der in (D.A., Hopwood et al., s.o. S. 79) beschriebenen Methode isoliert, mit der Restriktionsendonuklease Sau3A unvollständig gespalten (s. Beispiel 7) und 2,8 kb bis 15 kb Fragmente nach Auftrennung auf einem 0,7 % Agarosegel isoliert (s. Beispiel 21).

Das nach Beispiel 26 isolierte Vektorplasmid pEB15 wird mit der Restriktionsendonuklease BglII vollständig gespalten (s. Beispiel 1) und nach Beispiel 2 dephosphoryliert. Für die anschließende Ligase-Reaktion (s. Beispiel 3) werden 0,1 μg der isolierten Sau3A-Fragmente von S. coelicolor mit 0,05 μg BglII geschnittener, dephosphorylierter pEB15-DNA eingesetzt.

Der Ligase-Ansatz wird anschließend für die Transformation von Protoplasten der gemäß Beispiel 24 isolierten Lysozym defizienten Mutante, S. coelicolor DSM 4913 nach der in Beispiel 25 beschriebenen Methode eingesetzt.

## Beispiel 28

Klonierung von 3,3 kb SmaI-DNA-Fragmenten von S. coelicolor DSM 3030 in die Lysozym defiziente Mutante DSM 4913.

Das durch die Lysozymgen-spezifische 38 kb Oligonukleotidsonde (s. Beispiel 10) identifizierte 3,3 kb SmaI-Fragment der Gesamt-DNA von S. coelicolor DSM 3030 wurde nach der in Beispiel 21 beschriebenen Methode isoliert.

Der Vektor pGM4 (EP 0257417) wird mit der Restriktionsendonuklease BamHI vollständig gespalten, die überhängenden Enden mit E. coli DNA-Polymerase I (Klenow-Fragment) aufgefüllt (Maniatis T., et al. s.o. S. 113), mit alkalischer Phosphatase behandelt (s. Beispiel 2) und mit isolierten 3,3 kb großen SmaI-Fragmenten der S. coelicolor-DNA durch T4-DNA Ligase verbunden (s. Beispiel 3). Der Ligase-Ansatz wird anschließend in Protoplasten von S. coelicolor DSM 4913 transformiert (s. Beispiel 25).

## Beispiel 29

Nachweis der Lysozymproduktion komplementierter Mutanten

Die gemäß Beispiel 27 oder Beispiel 28 erhaltenen S. coelicolor Klone werden auf Blöckchen aus SM-Nährmedium, das mit 30 μg/ml Thiostrepton supplementiert war, überimpft und nach fünf Tagen Bebrütung bei 30°C in einer feuchten Kammer auf einen Lysozymtestagar (s. Beispiel 23) umgesetzt. Nach 16 Stunden Inkubation bei 30°C in einer feuchten Kammer war bei jeweils einem von 1500 nach Beispiel 27 bzw. von 150 nach Beispiel 28 erhaltenen Klonen eine dem Wildtyp S. coelicolor DSM 3030 entsprechende Lysozymproduktion durch die Ausbildung eines trüben Lysehofes auf dem Micrococcus-Testagar zu erkennen.

**Beispiel 30**

Isolierung und Charakterisierung der Plasmide Lysozym-bildender Klone

Die Plasmide pCel1 bzw. pCel2 wurden aus den nach Beispiel 28 bzw. Beispiel 27 erhaltenen Lysozym-bildenden S. coelicolor Klonen nach der in Hopwood D.A., et al. s.o. S. 85 beschriebenen Methode isoliert und durch Restriktionskartierung charakterisiert (s. Fig. 3 und Fig. 4). Das Plasmid pCel1 (s. Fig. 3) enthält ein das Lysozym (lys) kodierende 3,3 kb Smal-Fragment der Gesamt-DNA von S. coelicolor DSM 3030 in den Vektor pGM4 inseriert, welcher mit BamHI geschnitten worden war, und dessen überstehende Enden anschließend aufgefüllt worden waren. Der Vektor pGM4 enthält das Thiostrepton-Resistenzgen (tsr) von S. azureus und das Neomyzin-Resistenzgen (aphl) von S. fradiae. Das Plasmid pCel2 (s. Fig. 4) enthält ein Lysozym (lys) kodierendes 2,9 kb Sau3A-Fragment der Gesamt-DNA von S. coelicolor DSM 3030 in den mit BglII gespaltenen Vektor pEB15 einkloniert. Vektor pEB15 enthält das Tyrosinasegen (mel) von S. antibioticus und das Thiostrepten-Resistenzgen (tsr) von S. azureus.

**Beispiel 31**

Übertragung Lysozym kodierender Plasmide in S. lividans und in verschiedene S. coelicolor Stämme

Die Plasmide pCel1 und pCel2 (s. Beispiel 30) wurden jeweils in die nach Beispiel 23 und Beispiel 24 erhaltenen Lysozym defizienten Mutanten von S. coelicolor DSM 3030 transformiert (s. Beispiel 25). In anschließenden Schüttelkulturtests (s. Beispiel 23) in SM-Nährlösung, die mit 50 m Mol/l Hepes, pH 7,0 und 30 μg/ml Thiostrepton versetzt wurde, wurde bei allen untersuchten Transformanden durch photometrischen Test (DE 3 440 735) eine dem Wildtyp, S. coelicolor DSM 3030 entsprechende Lysozymproduktion festgestellt.

Die beiden Plasmide konnten jeweils nach der von C. J. Thompson et al., [J. Bacteriol. 151, 668-677 (1982)] beschriebenen Methode in S. lividans Tk64 (Hopwood et al., s.o. S. 266) übertragen werden. Die isolierten Transformanden weisen jedoch weder in CaSo-Nährlösung (s. Beispiel 25) noch in SM-Nährlösung, die jeweils 50 m Mol/l Hepes, pH 7,0 und 30 μg/ml Thiostrepton enthielten, eine durch photometrischen Test (DE 3440735) nachweisbare Lysozymproduktion auf.

Wird dagegen eines der nach Beispiel 30 isolierten Plasmide pCel 1 oder pCel 2 in S. coelicolor DSM 3030 transformiert kann in anschließendem Schüttelkulturtest mit SM-Nährmedium, das mit 50 m Mol/l Hepes (pH 7,0) und 30 μg/ml Thiostrepton supplementiert war, eine 2-3 fach erhöhte Lysozymausbeute im Vergleich zum Ausgangsstamm festgestellt werden. In Abwesenheit des Antibiotikums Thiostrepton wird dieselbe Steigerung der Lysozymproduktion in SM-Medium erreicht.

**Beispiel 32**

Stabilitätsuntersuchungen

S. coelicolor DSM 3030, der das Plasmid pCel2 enthielt (s. Beispiel 30), wird in SM-Nährlösung mit 50 m Mol/l Hepes (pH 7,0) ohne Zugabe von Antibiotikum 10 Tage bei 30°C geschüttelt. Nach 3, 5, 7 und 10 Tagen werden jeweils Proben entnommen und in geeigneter Verdünnung auf jeweils fünf Nährbodenplatten mit SM-Nährboden mit bzw. ohne Zusatz von 30 μg/ml Thiostrepton verteilt. Nach sieben Tagen Bebrütung bei 30° C wurde jeweils derselbe Bakterientiter auf beiden Plattensorten festgestellt. Von SM-Nährboden-Platten ohne Thiostrepton werden jeweils 500 Kolonien auf SM-Nährboden-Platten mit 30 µg/ml Thiostrepton überimpft. Nach 7 Tagen Bebrütung bei 30° C konnte die gleiche Anzahl von Kolonien auf beiden Plattensorten festgestellt werden.

**Beispiel 33**

Klonierung der Promotorregion des Tyrosinasegens von Streptomyces antibioticus vor das Lysozymgen von S. coelicolor DSM 3030.

Das nach Beispiel 30 isolierte Plasmid pCel2 (s. Fig. 4) wird mit Restriktionsendonuklease Aval vollständig gespalten, und die überstehenden Enden mit E. coli DNA-Polymerase I (Klenow-Fragment) aufgefüllt (Maniatis T. et al., s.o., S. 113). Das erhaltene 1,2 kb DNA-Fragment wird nach der in Beispiel 21 beschriebenen Methode isoliert. Das Vektorplasmid pEB15 (s. Beispiel 26) wird mit dem Enzym BglII vollständig gespalten, die überstehenden Enden aufgefüllt, dephosphoryliert (s. Beispiel 2) und mit dem isolierten 1,2 kb großen DNA-Fragment von pCel2 ligiert (s. Beispiel 3). Der Ligase-Ansatz wird in S. coelicolor DSM 4913 transformiert (s. Beispiel 25) und die erhaltenen Transformanden auf Agarblöckchen mit SM-Nährboden (s. Beispiel 23), der mit 30 µg/ml Thiostrepton supplementiert war, überimpft und in einer feuchten Kammer fünf Tage bei 30° C bebrütet. Die Blöckchen werden anschließend auf eine Platte mit Micrococcus-Testagar (s. Beispiel 2) umgesetzt und weitere 16 Stunden bei 30° C in einer feuchten Kammer inkubiert. Von den Lysozym bildenden Transformanden, welche an der Ausbildung eines trüben Lysehofes auf dem Testagar erkannt werden, wird Plasmid-DNA nach der in D.A. Hopwood et al., s.o., S. 85, beschriebenen Methode isoliert und mit der Restriktionsendonuklease PvuII vollständig gespalten. Das Plasmid pCel3 (s. Fig. 4) wird isoliert, das ein 2,1 kb und ein 4,2 kb PvuII-Fragment aufweist. Durch die Größe der erhaltenen PvuII-Fragmente von pCel3 ist die Orientierung des Lysozymgens (lys) wie in Fig. 4 dargestellt festgelegt. Die Promotorregion des Tyrosinasegens (mel) ist dem Lysozymgen von pCel3 mit dessen Promotor und Signalsequenz vorangeschaltet. Das Plasmid pCel3 wird in S. coelicolor DSM 3030 transformiert (C. J. Thompson et al., 1982, J. Bacteriol. 151, 668-677). Die Lysozymproduktion der so erhaltenen Transformanden ist gegenüber dem Ausgangsstamm S. coelicolor DSM 3030 in Schüttelkulturversuchen (s. Beispiel 23) mit SM-Medium, das mit 50 m Mol/l Hepes, pH 7,0, supplementiert war, mit bzw. ohne Zusatz von 30 µg/ml Thiostrepton jeweils um das 3- bis 5-fache erhöht.

**Ansprüche**

1. Lysozymgen aus Streptomyceten, dadurch gekennzeichnet, daß es
a) mit einer Oligonukleotidsonde der Sequenz
5'-TTC GC (G/C)TAC ATC AAG GC(G/C) AC(G/C) GAG GG(G/C) AC(G/C) AAC TAC AA-3'
hybridisiert und/oder
b) die Lysozym defiziente Mutante Streptomyces coelicolor DSM 4913 nach Transformation komplementiert.
2. Lysozymgen nach Anspruch 1 aus Streptomyces labedae oder Streptomyces coelicolor (Müller).
3. Lysozymgen nach Anspruch 2 aus S. labedae DSM 41517 oder S. coelicolor DSM 3030.
4. Lysozymgen nach einem oder mehreren der Ansprüche 1 bis 3, dadurch gekennzeichnet, daß es auf einem Aval-Fragment mit 1,2 kb lokalisiert ist.
5. Lysozymgen nach Anspruch 4, dadurch gekennzeichnet, daß es die in Tabelle 1 aufgeführte Sequenz hat.
6. Lysozymgen mit der in Tabelle 2 dargestellten Sequenz.
7. Lysozymgen nach Anspruch 6, dadurch gekennzeichnet, daß heterologe Kontroll- oder Signalsequenzen vor- oder nachgeschaltet sind.
8. Lysozymgen nach Anspruch 7, dadurch gekennzeichnet, daß die Promotorregion des Tyrosinasegens von Streptomyces antibioticus vorgeschaltet wird.
9. Hybridvektor enthaltend das Lysozymgen nach einem oder mehreren der Ansprüche 1 bis 8.
10. Mikroorganismus enthaltend einen Hybridvektor nach Anspruch 9.
11. Lysozym exprimiert von einem Mikroorganismus nach Anspruch 10.
12. Lysozym-defiziente Mutante Streptomyces coelicolor DSM 4913.

13. Oligonukleotidsonde der Sequenz

5'-TTC GC (G/C)TAC ATC AAG GC(G/C) AC(G/C) GAG GG(G/C) AC(G/C) AAC TAC AA-3'

14. S. coelicolor DSM 4914 enthaltend das Plasmid pEB15.

# Fig. 1    RESTRIKTIONSKARTE DES LYSOZYMGEN-BEREICHS

NUR AUSGEWÄHLTE SCHNITTSTELLEN SIND ANGEGEBEN

EP 0 368 224 A2

# 𝔽𝔦𝔤. 2   LYSOZYMGEN – SEQUENZIERUNGEN

**KLON 1**

Bss HII    Apa I    Sst II/Not I    Apa I    Sst I    Mlu I    Bss HII    Pvu II    Sal I    Ava I

Sma I

- - - -9503 - - - →
- - - -9507 - - - →
- - -9502 - - - -
← - - - -9504 - - - -
← - - - -9508 - - - - - -
← - - - -9501 - - -
← -9507 - - -
- - - -9506 - - →
- - -9509 - →

**KLON 2**

Bss HII    Apa I    Sst II/Not I    Apa I    Sst I    Mlu I    Bss HII    Pvu II    Sal I    Ava I

Sma I

p AB 21
p AB 18
p AB 12
p AB 19
p AB 15
p AB 20
p AB 17
p AB 11
p AB 14

EP 0 368 224 A2

## Fig. 3

SmaI  NotI                     NotI                        SmaI

lys                                                        pCel1

Bam HI
4.45
Sst I 4.55
4.25 Pst I
Nco I 4.7
4.2  Sst I
Sst II 4.75
4.15 Nco I

aph I

Kpn I 0.7
Bcl I 0.8

3.85 Xba I
4.0
3.75 Sst II

pGM 4
4.75 kb

3.4 Sst II

1.0
Pst I 1.0

3.35 Cla I
tsr
Sst II 1.2

3.0
3.05 Eco RV

2.0

2.8 Pvu II
Pst I 1.9

Sph I 2.1

# Fig. 4